# EUROPÄISCHE PATENTSCHRIFT

(11) **EP 1 648 870 B1**
(45) Veröffentlichungstag und Bekanntmachung des Hinweises auf die Patenterteilung: **19.10.2011**
(21) Anmeldenummer: 04763083.5
(22) Anmeldetag: 02.07.2004
(51) Int. Cl.: C07D 215/12, G01N 33/58, C07D 215/14, C07D 215/20, C07D 215/38, C07D 401/12, C07D 405/12, C07D 221/18, C07D 471/04, C07D 491/04, C07D 491/14, C07D 491/22

(54) **SULFONSÄUREDERIVATE POLYCYCLISCHER FARBSTOFFE FÜR ANALYTISCHE ANWENDUNGEN**
SULFONIC ACID DERIVATIVES OF POLYCYCLIC DYES USED FOR ANALYTICAL APPLICATIONS
DERIVES SULFONAMIDE DE COLORANTS POLYCYCLIQUES POUR APPLICATIONS ANALYTIQUES

(30) Priorität: 02.07.2003 DE 10329860
(43) Veröffentlichungstag der Anmeldung: 26.04.2006
(60) Teilanmeldung: 11151145.7
(73) Patentinhaber: ATTO-TEC GmbH, 57076 Siegen (DE)
(72) Erfinder: ZILLES, Alexander, 55490 Mengerschied (DE); ARDEN-JACOB, Jutta, 90513 Zirndorf (DE); DREXHAGE, Karl-Heinz, 57076 Siegen (DE); KEMNITZER, Norbert, Uwe, 57250 Netphen (DE); HAMERS-SCHNEIDER, Monika, 57258 Freudenberg (DE)
(74) Vertreter: Dey, Michael
(86) Internationale Anmeldenummer: PCT/EP2004/007248
(87) Internationale Veröffentlichungsnummer: WO 2005/003086

(56) Entgegenhaltungen:
- WO-A-87/00048
- WO-A-88/08420
- WO-A-92/07833
- WO-A-99/15517
- WO-A2-02/099077
- GB-A- 2 044 258
- GB-A- 2 054 582
- GB-A- 2 148 917
- GB-A- 2 148 918
- GB-A- 2 148 919
- GB-A- 2 148 920
- PANCHUK-VOLOSHINA, NATALIYA ET AL: "Alexa dyes, a series of new fluorescent dyes that yield exceptionally bright, photostable conjugates" JOURNAL OF HISTOCHEMISTRY AND CYTOCHEMISTRY , 47(9), 1179-1188 CODEN: JHCYAS; ISSN: 0022-1554, 1999, XP002360485
- DATABASE CAPLUS [Online] CHEMICAL ABSTRACTS SERVICE, COLUMBUS, OHIO, US; BAR, VILMOS ET AL: "Dihydroquinolinemethanesulfonic acid derivatives" XP002360487 gefunden im STN Database accession no. 1986:497341 -& HU 188 100 B (MATERIAL VEGYIPARI SZOVETKEZET, HUNG.) 28. März 1986 (1986-03-28)

## Beschreibung

Die Erfindung betrifft die Herstellung von Sulfonsäuregruppen-haltigen Chinolinverbindungen, die Chinolinverbindungen selbst sowie deren Umsetzung zu Sulfonsäuregruppen-enthaltenden Farbstoffen. Die erfindungsgemäßen Farbstoffe finden insbesondere Anwendung zur Markierung von Analyten, beispielsweise zur Markierung von Biomolekülen.

Farbstoffe aus der Klasse der Cumarin-, der Xanthen-, der Oxazinfarbstoffe sowie verwandte Derivate werden aufgrund ihrer sehr guten spektralen Eigenschaften, insbesondere ihrer Fluoreszenz, bevorzugt in der chemischen, biologischen und medizinischen Analytik als Markierungsgruppen eingesetzt (J. Slavik, Fluorescent Probes in Cellular and Molecular Biology, CRC Press, Boca Raton, Ann Arbor, London, Tokyo, 1994). Eine große Anzahl von langwelligen Xanthen-Farbstoffen (Rhodamine etc.) werden beschrieben in EP 0567622 B1. Markierungsfarbstoffe aus der Klasse der Oxazine werden offenbart in EP 0747447 B1. Sulfonsäurederivate von Xanthen-Farbstoffen sind beschrieben in WO 99/15517. Farbstoffe aus den Klassen der Carbopyronine und Amidopyrylium-Farbstoffe werden beschrieben in WO 00/64986 und WO 00/64987. Dabei spielen vor allem Farbstoffe mit einer hohen Fluoreszenzquantenausbeute eine wichtige Rolle, da über die Fluoreszenz ein sehr empfindlicher Nachweis des markierten Analyten möglich ist. Aber auch nicht-fluoreszierende Derivate gewinnen zunehmend als Quencher in speziellen Verfahren Bedeutung.

Zur Verwendung als Markierungsgruppen für Analyte ist neben einer einfachen und zuverlässigen Nachweisbarkeit eine gute Löslichkeit, z.B. in wässrigen Systemen, erforderlich. In anderen Fällen ist, gerade umgekehrt, gute Löslichkeit in unpolarer Umgebung, z.B. Zellmembranen, erwünscht.

Von entscheidender Bedeutung für die Anwendbarkeit als Markierungsgruppen für Analyte oder in ähnlichen Verfahren sind also je nach den konkreten Bedingungen bestimmte spezifische Eigenschaften, über die der Fluorophor zusätzlich zu seinen optischen Eigenschaften verfügen sollte. Diese sind bei den Farbstoffen der genannten Patente entweder gar nicht oder, wenn überhaupt, nur sehr umständlich durch Farbstoffsynthese unter Verwendung entsprechender Edukte zu erreichen.

Panchuk-Voloshina et al. (J. Histochem. Cytochem. (1999), 47, 1179-1188) beschreibt die Herstellung polycyclischer Farbstoffe sowie deren Verwendung zur Erzeugung fluoreszierender, photostabiler Konjugate. Im Einzelnen wird hierbei u.a. der tricyclische Farbstoff Alexa 430 erwähnt, welcher durch Sulfonierung des korrespondierenden Aminocumarins erhalten werden kann.

WO 02/099077 A2 offenbart Zusammensetzungen und Verfahren zur Markierung von Membranproteinen, wobei als Markierungsreagenz polycyclische Farbstoffe zur Anwendung gelangen. Im Einzelnen wird in diesem Zusammenhang der tricyclische Farbstoff Cumarin 430 beschrieben, welcher eine 1,2-Dihydrochinolin-Einheit mit einer hieran gebundenen Methylsulfonsäuregruppe umfasst.

Eine Aufgabe der vorliegenden Erfindung bestand deshalb darin, bestimmte physikalische und/oder chemische Eigenschaften - etwa die Löslichkeit in bestimmten Medien, die Tendenz zu unspezifischen Wechselwirkungen mit Substraten und/oder Gefäßwänden oder Kopplungsfähigkeit - dieser Farbstoffe in einfacher Weise - auch nachträglich - zu modifizieren, ohne die sehr guten spektralen Eigenschaften der Farbstoffe wesentlich zu verändern.

Diese Aufgabe wird erfindungsgemäß gelöst durch die Darstellung von Verbindungen der allgemeinen Formeln Ia und Ib

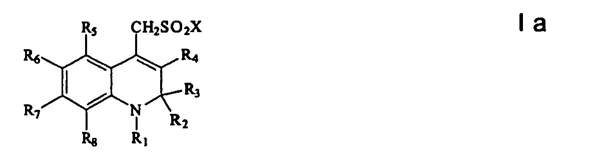

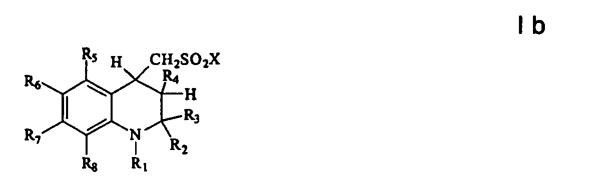

Sowie ihre Verwendung zur Darstellung von Farbstoffen, die z.B. als Markierungsgruppen in einem Verfahren zum Nachweis von Analyten eingesetzt werden können, wobei
R₁ eine gesättigte oder ungesättigte, geradkettige, verzweigte oder cyclische Kohlenwasserstoffgruppe mit bis zu 20 C-Atomen, z.B. Polyether, Phenyl, Phenylalkyl mit 1-3 C-Atomen in der Kette bedeuten, wobei die Kohlenwasserstoffgruppen gegebenenfalls Heteroatome, wie Sauerstoff-, Schwefel- oder Stickstoffatome, oder/und einen oder mehrere Substituenten, vorzugsweise ausgewählt aus Halogenen, Hydroxy-, Amino-, Sulfo-, Phospho-, Carboxy-, Carbonyl-, Alkoxy- oder/und Alkoxycarbonylgruppen, enthalten können,
R₂, R₃, R₄, R₅, Re, R₇ und R₈ jeweils unabhängig Wasserstoff, Halogen, eine Hydroxy-, Amino-, Sulfo- oder Carboxy- oder Aldehydgruppe oder eine gesättigte oder ungesättigte, geradkettige, verzweigte oder cyclische Kohlenwasserstoffgruppe mit bis zu 20 C-Atomen bedeuten, wobei die Kohlenwasserstoffgruppen Alkyl-, Alkenyl-, Alkinyl-, Cycloalkyl-, Aryl, insbesondere Phenyl-, oder/und Heteroarylreste umfassen und gegebenenfalls auch Heteroatome, wie Sauerstoff-, Schwefel- oder Stickstoffatome, oder/und mehrere Substituenten, vorzugsweise ausgewählt aus Halogenen, Hydroxy-, Amino-, Phospho-, Sulfo-, Carboxy-, Carbonyl-, Alkoxy- oder/und Alkoxycarbonylgruppen, enthalten können, oder der Rest R₈ mit R₁ ein Ringsystem bilden, das eine oder mehrere Mehrfachbindungen enthalten kann, und
X Alkylthio- oder Aminogruppe bedeuten, wobei die gegebenenfalls vorhandenen Kohlenwasserstoffreste dieser Gruppen insbesondere Alkyl-, Alkenyl-, Alkinyl-, Cycloalkyl-, Aryl, insbesondere Phenyl-, oder/und Heteroarylreste umfassen und gegebenenfalls auch Heteroatome, wie Sauerstoff-, Schwefel- oder Stickstoffatome, oder/und mehrere Substituenten, vorzugsweise ausgewählt aus Halogenen, Hydroxy-, Amino-, Phospho-, Sulfo-, Carboxy-, Carbonyl-, Alkoxy- oder/und Alkoxycarbonylgruppen, enthalten können.

Bei bevorzugten Ausführungsformen bedeutet R₁ einen Aryl- oder Alkylrest, R₂ und R₃ jeweils Methyl und R₄ Wasserstoff.

In weiteren besonders bevorzugten Formen bedeutet R₁ einen Aryl- oder Alkylrest, R₂ und R₃ jeweils Methyl, R₄ Wasserstoff und R₇ eine Hydroxyoder Methoxygruppe.

In noch weiteren besonders bevorzugten Formen bedeutet R₁ einen Aryloder Alkylrest, R₂ und R₃ jeweils Methyl, R₄ Wasserstoff und R₆ eine Nitroso-, Formyl- oder eine Hydroxymethylgruppe.

In einer weiteren besonders bevorzugten Verbindungsklasse sind R₁ mit R₈ verbrückt und bilden ein Ringsystem, insbesondere einen 5- oder 6-gliedrigen Ring.

Die vorzugsweise verwendeten bzw. dargestellten Farbstoffe sind Vertreter der nachfolgend zusammengestellten Klassen - aber nicht auf diese Klassen beschränkt- der allgemeinen Formeln II - VII. Bei Farbstoffen der Klassen III und V - VII, die über ein symmetrisches chromophores System verfügen, ist es erfindungsgemäß durch Wahl der Edukte möglich, zwei - gleiche oder verschiedene - SO₂X-Gruppen einzuführen. Durch nachträgliche Hydrierung ist es in gleicher Weise wie bei den Edukten I möglich, entsprechende Farbstoffe mit Tetrahydrochinolin-Endgruppen herzustellen.

| | | |
|---|---|---|
| 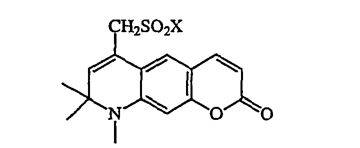 | II | Cumarine |
| 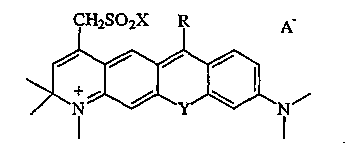 | III | Xanthenfarbstoffe Y = O, S, Se |
| 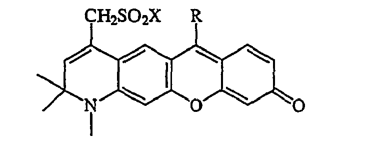 | IV | Rhodol-Farbstoffe |
| 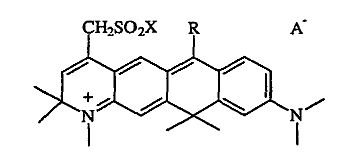 | V | Carbopyronine |
| 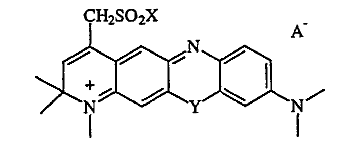 | VI | Azin-Farbstoffe Y = O (Oxazine), S (Thiazine), CR₂ (Carbazine) |
| 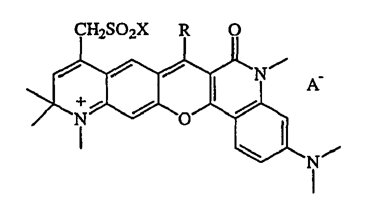 | VII | Amidopyryliumfarbstoffe |

wobei R insbesondere = H, (subst.) Aryl, (subst.) Carboxyphenyl, (subst.) Alkyl, (subst.) Cycloalkyl, (subst.) P-yridyl, (subst.) Carboxypyridyl etc. Erfindungsgemäß können entweder entsprechende Synthesevorstufen oder die eigentlichen Farbstoffe sulfoniert und anschließend die Sulfonsäuregruppe(n) derivatisiert werden. Dazu wird die Sulfonsäure oder ihr Salz bevorzugt in ein Sulfochlorid überführt und dieses anschließend mit einem Nucleophil (z.B. Amino- oder Mercaptogruppe) umgesetzt. Die Sulfonierung gelingt z.B. in konzentrierter Schwefelsäure, gegebenenfalls im Gemisch mit Oleum. Die Sulfonsäurederivate sowohl der Vorstufen als auch der Farbstoffe lassen sich in einfacher Weise mit Phosphoroxychlorid, Phosphorpentachlorid oder Thionylchlorid bevorzugt im Gemisch mit Dimethylformamid zum Sulfochlorid umsetzen. Überraschenderweise können die Sulfochloride in fast allen Fällen problemlos isoliert werden. Die Sulfochloride reagieren mit verschiedenen Nucleophilen, vorzugsweise mit primären oder sekundären Aminen, zu entsprechenden Sulfonamid-Derivaten. Die physikalischen und/oder chemischen Eigenschaften der so erhaltenen Verbindungen hängen dabei auch ganz entscheidend von der Natur des eingesetzten Amins ab, wie später noch ausführlicher - auch anhand von Beispielen - erläutert wird. Dabei wird eine erstaunlich große Vielfalt von pH-stabilen Sulfonamid-Derivaten erhalten. Die erfindungsmäßige Art der Sulfonamid-Verknüpfung sorgt für eine räumliche Separation von Substituent und Chromophor und verhindert dadurch einen nachteiligen Einfluss auf die Fluoreszenz-Effizienz. Die Farbstoffsulfochloride können auch - ohne vorherige Derivatisierung - direkt zur Kopplung an nucleophile (NH₂ etc. Gruppen) Gruppen von Analytmolekülen verwendet werden.

Der Begriff Heteroatome, wie hierin verwendet, umfasst insbesondere Sauerstoff-, Schwefel- und Stickstoffatome.

Der Begriff Substituenten umfasst, soweit nicht anders angegeben, insbesondere Halogen, Hydroxy-, Amino-, Sulfo-, Phospho-, Carboxy-, Alkoxy- sowie Alkoxycarbonylgruppen mit, soweit vorhanden, 1-10 C-Atomen.

Zur Darstellung der erfindungsgemäßen Verbindungen werden folgende allgemeine Verfahren angewendet. Es können entweder die Vorstufen (Punkt 1) oder die fertigen Farbstoffe (Punkt 2) derivatisiert werden.

### 1. Synthese entsprechender Vorstufen

Ausgehend von einem nach literaturbekannten Methoden, z.B. durch die Kondensation eines Anilinderivats mit Mesityloxid und anschließende N-Alkylierung, dargestellten 7-Alkoxy-2,2,4-trimethyl-1,2-dihydrochinolin wird erfindungsgemäß folgendermaßen vorgegangen:

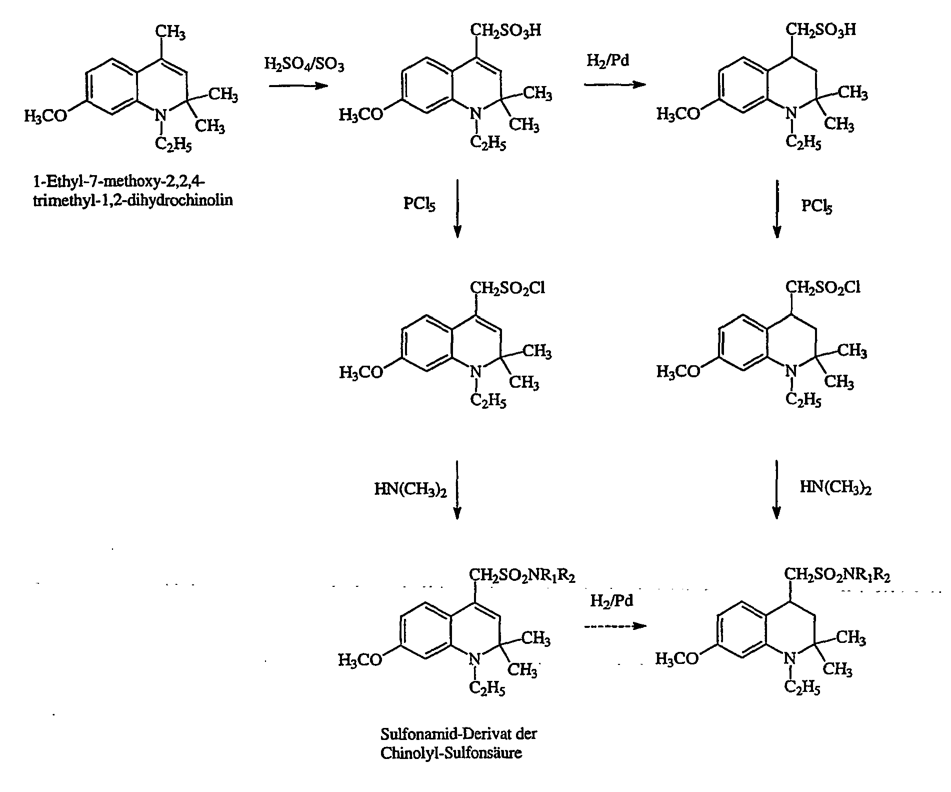

### Abbildung 1:

Beispielhafte Darstellung erfindungsgemäßer Verbindungen. Es wird ausgehend von 1-Ethyl-7-methoxy-2,2,4-trimethyl-1,2-dihydrochinolin über die Zwischenstufen der Sulfonsäure und des Sulfochlorids schließlich ein Sulfonamid-Derivat erhalten. Durch intermediäre Hydrierung lässt sich das entsprechende 1,2,3,4-Tetrahydrochinolin-Derivat erhalten.

Durch Verrühren des Dihydrochinolins mit einem Gemisch aus konzentrierter Schwefelsäure und Oleum bei Raumtemperatur erhält man ein an der 4-Methylgruppe sulfoniertes Produkt. Diese Sulfonsäure oder ihr Salz kann leicht in das Sulfochlorid überführt werden. Dazu wird die sulfonierte Verbindung bevorzugt bei Raumtemperatur in Benzol mit Phosphorpentachlorid umgesetzt. Allgemein verwendet man für die Darstellung des Sulfochlorids gebräuchliche Reagenzien wie Phosphoroxychlorid, Phosphorpentachlorid oder Thionylchlorid. Die Reaktion wird in einem wasserfreien inerten Lösungsmittel, vorzugsweise Benzol, ausgeführt.

Das Sulfochlorid kann durch einfache Umsetzung mit fast beliebigen primären und sekundären Aminen in ein entsprechendes Sulfonamid überführt werden. Umsetzungen des Sulfochlorids mit anderen Nucleophilen (Thiole, Alkohole etc.) sind ebenfalls möglich. Gegebenenfalls kann das erhaltene stabile Sulfonamid weiter derivatisiert werden (Verseifung von Carbonestergruppen o.ä.).

Das 1,2-Dihydrochinolin lässt sich z.B. auf der Stufe der Sulfonsäure palladiumkatalysiert mit Wasserstoff hydrieren und so in ein 1,2,3,4-Tetrahydrochinolin überführen. Dieses hydrierte Chinolin kann anschließend dem gleichen Syntheseweg unterworfen werden (siehe Abbildung 1).

Mit den so erhaltenen Sulfonsäuren, ihren Salzen oder bevorzugt den Sulfonamid-Derivaten werden die nachfolgenden Farbstoffsynthesen nach den dem Fachmann bekannten Darstellungsmethoden durchgeführt:

Symmetrische Rhodaminfarbstoffe lassen sich z.B. durch die Kondensation des beschriebenen Sulfonamid-Derivats mit Phthalsäureanhydrid erhalten. Andere Xanthenfarbstoffe, wie z.B. Trifluormethyl-, Pyronin- oder Rosaminfarbstoffe, erhält man durch Kondensation mit den entsprechenden Säureanhydriden (Trifluoressigsäureanhydrid) oder Aldehyden (Benzaldehyd etc.).

Unsymmetrische Farbstoffe erhält man im Falle der Rhodamine z.B. durch die Umsetzung von Phthalsäureanhydrid mit einem Aminophenol oder hydriertem 7-Hydroxy- bzw. 7-Methoxychinolin und die anschließende Umsetzung des gebildeten Benzophenon-Derivats, z.B. mit einem zweiten Dihydro- oder Tetrahydrochinolin, wobei jetzt etwa ein erfindungsgemäßes Sulfonamid-Derivat zum Einsatz kommen kann.

Die Vertreter anderer Farbstoffklassen, wie z.B. Cumarine, Carbopyronine, Amidopyryliumfarbstoffe, Oxazine usw., lassen sich mit den Sulfonsäuren, ihren Salzen oder den Sulfonamid-Derivaten der erwähnten oder ähnlicher Chinolinvorstufen nach literaturbekannten Methoden herstellen. Das heißt, in den bekannten Syntheseverfahren können die erfindungsgemäßen Verbindungen überraschenderweise problemlos für die Darstellung der Farbstoffe eingesetzt werden.

### 2. Nachträgliche Derivatisierung der Farbstoffe

Erfindungsgemäß können Farbstoffe nachträglich an der 4-Methylgruppe sulfoniert werden. Ein ähnliches Verfahren ist in der Patentanmeldung WO 99/15517 für Xanthenfarbstoffe beschrieben. Überraschenderweise gelingt eine solche nachträgliche Sulfonierung auch bei vielen anderen polycyclischen Farbstoffen. Dadurch vereinfacht sich die Synthese der erfindungsgemäßen Sulfonamidderivate beträchtlich.

Die so erhaltenen sulfonierten Farbstoffe sind erstaunlicherweise ebenfalls - wie oben im Falle der Edukte beschrieben - in Sulfochloride überführbar, ohne dass der Chromophor dabei verändert oder zerstört wird. Hier wird neben Phosphoroxychlorid und/oder Phosphorpentachlorid bevorzugt eine Mischung aus Thionylchlorid und Dimethylformamid verwendet. Die Sulfochloride können als Perchlorate isoliert und gereinigt werden. Die isolierten Farbstoffsulfochloride lassen sich in bekannter Weise direkt als Marker für Aminogruppen in Biomolekülen einsetzen. Die so gewonnenen Sulfochloride können aber auch - wie oben für die Chinolin-Vorstufen beschrieben - mit fast beliebigen primären und sekundären Aminen umgesetzt werden. Man kann auf diese Weise besonders einfach die erfindungsgemäßen Farbstoffe erhalten.

Durch die Einführung der Sulfonsäuregruppe und ihre Derivatisierung werden die spektralen Eigenschaften, wie Absorptions- und Fluoreszenzmaximum, Extinktionskoeffizient sowie die Fluoreszenzquantenausbeute der Farbstoffe, praktisch nicht beeinflusst.

Durch das beschriebene Verfahren kann man aber auf einfache Weise oder sogar nachträglich den Farbstoffen - je nach verwendetem primären oder sekundären Amin - eine Vielfalt neuer Eigenschaften vermitteln. Denn das Amin kann fast beliebige weitere funktionelle Gruppen tragen. So lassen sich z.B. Cyano-, Mercapto-, Halogen-, Sulfonsäure-, Hydroxy-, Alkenyl-Gruppen etc. durch unser Verfahren in den Farbstoff einführen.

Trägt das verwendete Amin z.B. längere Alkylketten, z.B. C₁₀ bis C₃₀, so erhöht sich der lipophile Charakter, und die "Verbindung ist in unpolaren Medien und Membranen löslich und kann so zum Nachweis von Membraneigenschaften oder zu molekularen Abstandsmessungen eingesetzt werden.

Die Wasserlöslichkeit eines Farbstoffs lässt sich verbessern, wenn das Amin wiederum Sulfon- oder Phosphonsäuregruppen trägt oder über Polyetherketten verfügt. Letztere machen die Verbindung auch in vielen organischen Lösungsmitteln besser löslich. Auch lassen sich dadurch ungewünschte unspezifische Wechselwirkungen mit Substrat oder Gefäßwänden vermindern. Eine Art cyclischer Polyether stellen die für den fluoreszenzsensitiven Kationennachweis verwendeten Kronenether dar, die als Aza-Derivate auch über die hier beschriebene Methode an Farbstoffmoleküle gekoppelt werden können. Ferner lassen sich nach dem beschriebenen Verfahren auch bichromophore Systeme darstellen, in denen Energieübertragung (FRET) zwischen den Chromophoren stattfindet.

Trägt das Amin Substituenten, die zur kovalenten Kopplung fähig sind, z.B. -COOH, -NH₂, -OH oder/und -SH, so kann die erfindungsgemäß hergestellte Verbindung nach bekannten Methoden an einen Träger und/oder an ein Biomolekül gekoppelt werden. Durch dieses Vorgehen können somit Farbstoffe zur Markierung verwendet werden, die an sich nicht über eine kopplungsfähige Carboxylgruppe o.ä. verfügen, wie es bei den in WO 99/15517 beschriebenen Sulfonsäurederivaten noch erforderlich ist. Als Träger kann jedes geeignete Material ausgewählt werden, z.B. poröses Glas, Kunststoffe, Ionenaustauscherharze, Dextrane, Cellulose, Cellulosederivate oder/und hydrophile Polymere. Die Biomoleküle werden vorzugsweise ausgewählt aus Peptiden, Polypeptiden, Nukleotiden, Nukleosiden, Nukleinsäuren, Nukleinsäureanaloga oder/und Haptenen.

Eine gängige Methode zur kovalenten Markierung von Biomolekülen ist die dem Fachmann vertraute Aktivestermethode. In einer bevorzugten Weise wird deshalb als Amin eine Carbonsäure mit terminaler Aminogruppe und mittlerer Kettenlänge eingesetzt. So lassen sich z.B. die käufliche 4-Methylaminobuttersäure, 4-Piperidincarbonsäure, 6-Aminohexansäure usw. als Amin in erfindungsgemäßer Weise einsetzen. Man erhält nach der Sulfonamidverknüpfung also Farbstoffe, die über eine koppelbare Carboxylgruppe verfügen. Nach der Ankopplung an primäre Aminogruppen geeigneter Biomoleküle lässt sich die verwendete Markierungsgruppe und damit der Analyt leicht durch ihre Absorption und/oder Fluoreszenz nachweisen.

Die erhaltenen Verbindungen können als Markierungsgruppen und damit der Analyt in Verfahren zur qualitativen oder/und quantitativen Bestimmung eines Analyten verwendet werden. Die Bestimmung kann in wässrigen Flüssigkeiten, z.B. Proben von Körperflüssigkeiten, wie etwa Blut, Serum, Plasma oder Urin, Abwasserproben oder Lebensmitteln, durchgeführt werden. Das Verfahren kann sowohl als Nasstest, z.B. in einer Küvette, oder als Trockentest auf einem entsprechenden Reagenzträger durchgeführt werden. Die Bestimmung der Analyten kann dabei über eine einzige Reaktion oder durch eine Sequenz von Reaktionen erfolgen. Die Verwendung der erhaltenen Verbindungen zeigt dabei sehr gute Ergebnisse in chemischen und insbesondere in medizinischen und biologischen Nachweisverfahren zur Bestimmung eines Analyten.

Die Verbindungen können in allen bekannten chemischen, medizinischen und biologischen Nachweisverfahren, in denen Fluoreszenzfarbstoffe als Markierungsgruppen geeignet sind, verwendet werden. Derartige Verfahren sind dem Fachmann bekannt und müssen deshalb nicht weiter ausgeführt werden.

In einer besonders bevorzugten Ausführungsform wird die erhaltene Verbindung kovalent an einen für den nachzuweisenden Analyten spezifischen Rezeptor gekoppelt. Der spezifische Rezeptor ist jede geeignete Verbindung oder jedes geeignete Molekül, vorzugsweise ist es ein Peptid, Polypeptid oder eine Nukleinsäure. Die Verbindungen oder Konjugate dieser Verbindungen können beispielsweise in Nukleinsäure-Hybridisierungsverfahren oder immunchemischen Verfahren verwendet werden. Derartige Verfahren sind beispielsweise beschrieben in Sambrook et al., Molecular Cloning, A Laboratory Manual, 1989, Cold Spring Harbor.

Ein wesentlicher Vorteil des beschriebenen Verfahrens ist die uneingeschränkte Anwendbarkeit auf alle Farbstoffe, für deren Synthese die oben beschriebenen Di- und Tetrahydrochinoline verwendet werden können. Man ist also in der Lage, durch die Auswahl des Chromophors genau die Farbstoffe auszuwählen, die aufgrund ihrer spektroskopischen Eigenschaften, der Lage der Absorptions- und Fluoreszenzmaxima, der Löslichkeitseigenschaften, der Fluoreszenzabklingzeit und der Höhe der Quantenausbeute für die gewünschte Anwendung besonders geeignet erscheinen.

Die Herstellung von erfindungsgemäßen Verbindungen gelingt nach den vorne beschriebenen Methoden in einfacher und kostenaünstiger Weise, wie in den nachfolgenden Beispielen erläutert wird. Die so hergestellten Verbindungen sind unproblematisch handhabbar und zeigen eine gute Stabilität und Lagerfähigkeit.

Die Erfindung betrifft somit insbesondere die Herstellung und Verwendung von Dihydrochinolin- und Tetrahydrochinolin-Derivaten entsprechend Formeln Ia und Ib mit X = NR₁R₂ mit R₁ und R₂= substituiertes Alkyl, Aryl etc. (Sulfonamide); X = SR mit R = Alkyl, Aryl etc. (Thiosulfonsäure-S-Ester), sowie die Verwendung von Verbindungen entsprechend Formeln Ia und Ib zur Herstellung polycyclischer Farbstoffe entsprechend Formeln II - VII.

Die Erfindung umfasst weiterhin die Herstellung und Verwendung polycyclischer Farbstoffe entsprechend Formeln II - VII durch direkte Einführung von ein oder mehreren Substituenten SO₂X in bekannte Farbstoffe mit Dihydrochinolin- oder Tetrahydrochinolin-Endgruppen.

Die Farbstoffe entsprechend Formeln II - VII werden vorzugsweise zur Markierung von Analyten, insbesondere Biomolekülen (Peptide, Nucleotide etc.), über deren NH₂- bzw. SH-Gruppen eingesetzt.

Die Erfindung umfasst weiterhin die Verwendung der Farbstoffe entsprechend Formeln II - VII mit X = NR₁R₂ und R₁ = COOH-substituiertes Alkyl oder Aryl zur Bildung von Aktivestern (mit z.B. N-Hydroxysuccinimid) und nachfolgende Kopplung an Aminogruppen eines Analyten. Zudem können die Farbstoffe entsprechend Formeln II - VII zur Kopplung an freie Aminogruppen anderer Farbstoffe (FRET-Pärchen) verwendet werden.

Konkrete Beispiele für erfindungsgemäße Verbindungen sind in den Tabellen 1 bis 3 dargestellt.

**Tabelle 1:**

| | | | |
|---|---|---|---|
| **Chinolylsulfonsäuren und deren Derivate** | | | |

| Beispiel | Struktur | Summenformel | Masse MH⁺ |
|---|---|---|---|
| **1** | nicht erfindungsgemäß | C₁₉H₂₇NO₆S | 398 |
| | 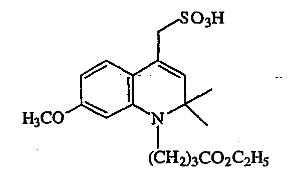 | | |
| **2** | nicht erfindungsgemäß | C₁₉H₂₉NO₆S | 400 |
| | 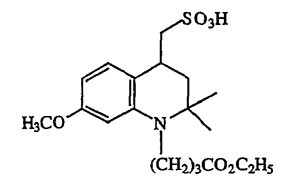 | | |
| **3** | nicht erfindungsgemäß | C₁₄H₂₁NO₄S | 300 |
| | 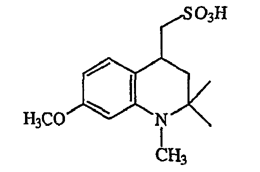 | | |
| **4** | nicht erfindungsgemäß | C₁₃H₁₉NO₄S | 286 |
| | 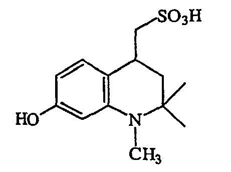 | | |
| **5** | nicht erfindungsgemäß | C₁₃H₁₈N₂O₅S | 315 |
| | 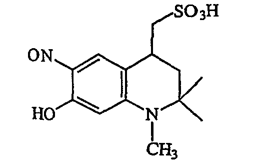 | | |
| **6** | nicht erfindungsgemäß | C₁₇H₂₇NO₇S₂ | 421 |
| | 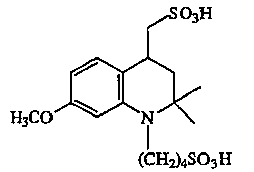 | | |
| **7** | nicht erfindungsgemäß | C₁₃H₁₉NO₄S | 286 |
| | 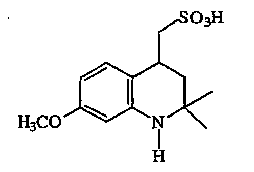 | | |
| **8** | nicht erfindungsgemäß | C₁₄H₁₉NO₃S | 284 |
| | 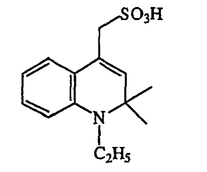 | | |
| **9** | nicht erfindungsgemäß | C₁₄H₁₈ClNO₂S | 302 |
| | 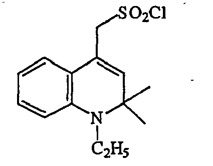 | | |
| **10** | 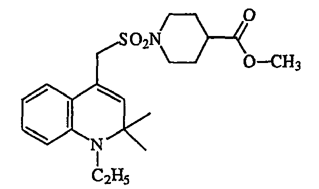 | C₂₁H₃₀N₂O₄S | 407 |
| **11** | 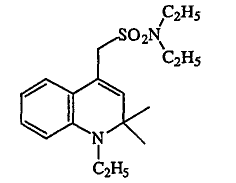 | C₁₈H₂₈N₂O₂S | 337 |
| **12** | 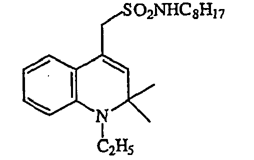 | C₂₂H₃₆N₂O₂S | 393 |
| **13** | 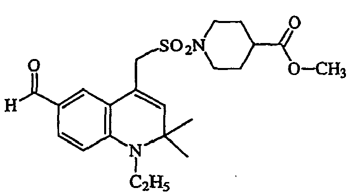 | C₂₂H₃₀N₂O₅S | 435 |
| **14** | 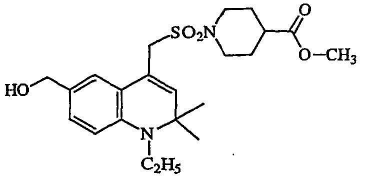 | C₂₂H₃₂N₂O₅S | 437 |
| **15** | nicht erfindungsgemäß | C₁₅H₂₁NO₄S | 312 |
| | 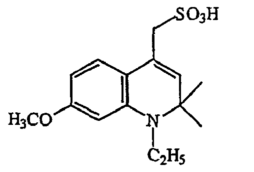 | | |
| **16** | nicht erfindungsgemäß | C₁₅H₂₂CINO₃S | 330 |
| | 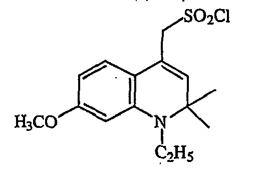 | | |
| **17** | 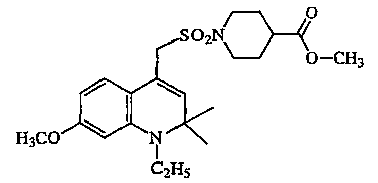 | C₂₂H₃₂N₂O₅S | 437 |
| **18** | nicht erfindungsgemäß | C₁₄H₁₉NO₄S | 298 |
| | 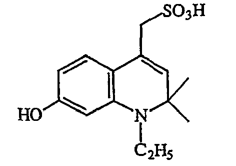 | | |
| **19** | nicht erfindungsgemäß | C₁₄H₂₀CINO₃S | 318 |
| | 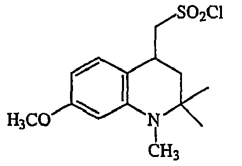 | | |
| **20** | 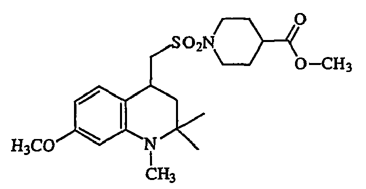 | C₂₁H₃₂N₂O₅S | 425 |
| **21** | 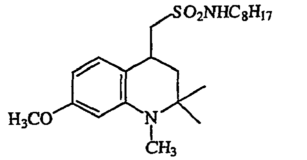 | C₂₂H₃₈N₂O₃S | 411 |
| **22** | 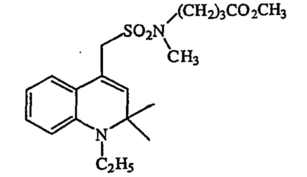 | C₂₀H₃₀N₂O₄S | 395 |
| **23** | 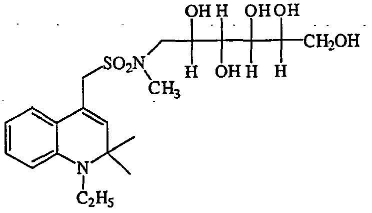 | C₂₁H₃₄N₂O₄S | 459 |
| **24** | 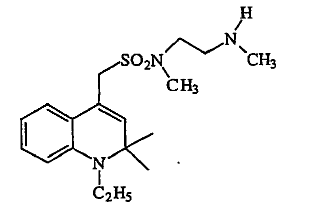 | C₁₈H₂₉N₃O₂S | 352 |
| **25** | 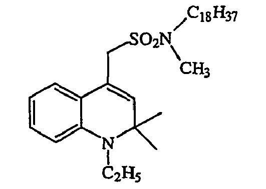 | C₃₃H₅₈N₂O₂S | 547 |
| **26** | 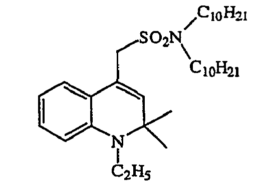 | C₃₄H₆₀N2O₂S | 561 |
| **27** | 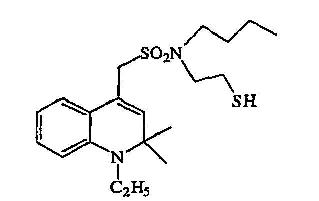 | C₂₀H₃₂N₂O₂S₂ | 397 |
| **28** | 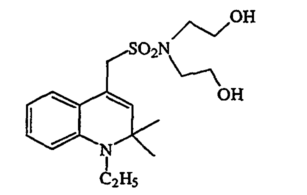 | C₁₈H₂₈N₂O₄S | 369 |
| **29** | 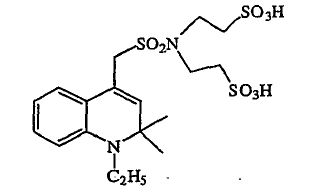 | C₁₈H₂₈N₂O₈S₃ | 497 |
| **30** | 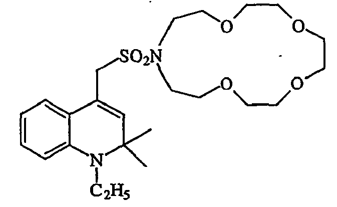 | C₂₄H₃₈N₂O₆S | 483 |
| **31** | 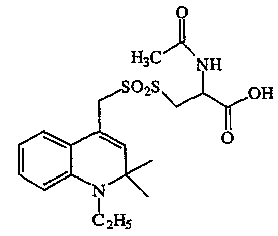 | C₁₉H₂₆N₂O₅S₂ | 427 |
| **32** | 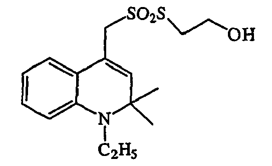 | C₁₆H₂₃NO₂S₂ | 341 |
| **33** | 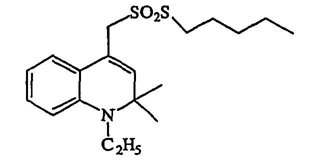 | C₁₉H₂₉NO₂S₂ | 367 |

**Tabelle 2a:**

| | | | |
|---|---|---|---|
| **Cumarinfarbstoffe mit Sulfonsäure-Gruppe** | | | |
| Spektrale Daten in Ethanol: | λₐ Absorptionsmaximum, | | |
| | λ_{f} Fluoreszenzmaximum | | |

| Name | Struktur | λₐ / nm | λ_{f}/ nm |
|---|---|---|---|
| **34** | nicht erfindungsgemäß | 394 | 468 |
| AZ 59 | 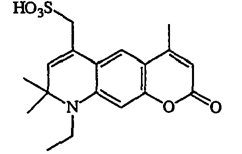 | | |
| **35** | nicht erfindungsgemäß | 383 | 455 |
| AZ 100 | 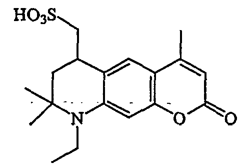 | | |
| **36** | nicht erfindungsgemäß | 394 | 469 |
| AZ 101 | 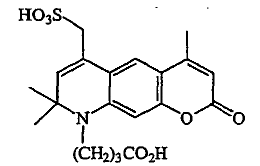 | | |

**Tabelle 2b:**

| | | | |
|---|---|---|---|
| **Rhodaminfarbstoffe mit Sulfonsäure-Gruppe** | | | |
| Spektrale Daten in Ethanol: | λₐ Absorptionsmaximum, | | |
| | λ_{f} Fluoreszenzmaximum | | |

| Name | Struktur (Anion A⁻) | λₐ/nm | λ_{f}/nm |
|---|---|---|---|
| **37** | nicht erfindungsgemäß | 592 | 612 |
| JA 317 | 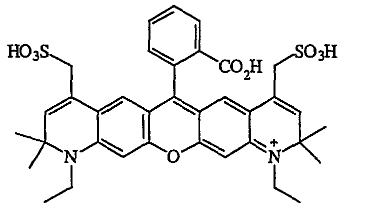 | | |
| **38** | nicht erfindungsgemäß | 621 | 642 |
| JA 325 | 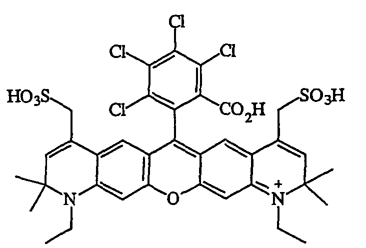 | | |
| **39** | nicht erfindungsgemäß | 562 | 587 |
| AZ 58 | 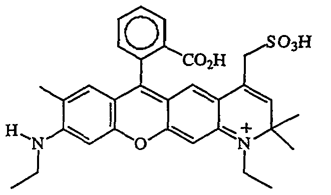 | | |
| **40** | nicht erfindungsgemäß | 623 | 645 |
| JA 407 | 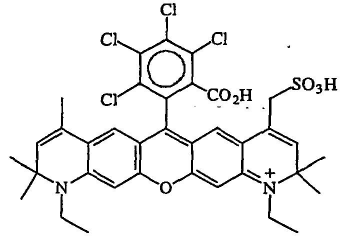 | | |
| **41** | nicht erfindungsgemäß | 623 | 644 |
| JA 407-E | 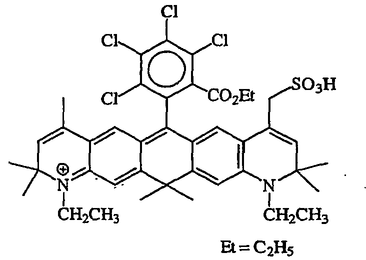 | | |

**Tabelle 2c:**

| | | | |
|---|---|---|---|
| **Oxazinfarbstoffe mit Sulfonsäure-Gruppe** | | | |
| Spektrale Daten in Ethanol: | λₐ Absorptionsmaximum, | | |
| | λ_{f} Fluoreszenzmaximum | | |

| Name | Struktur (Anion A⁻) | λₐ/nm | λ_{f}/nm |
|---|---|---|---|
| **42** | nicht erfindunsgsgemäß | 655 | 680 |
| JA 378 | 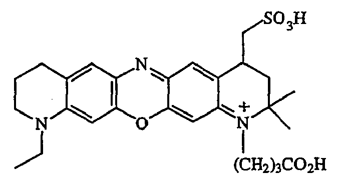 | | |
| **43** | nicht erfindunsgsgemäß | 675 | 699 |
| JA 379 | 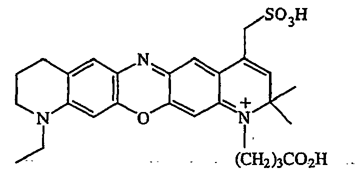 | | |
| **44** | nicht erfindunsgsgemäß | 674 | 699 |
| JA 322 | 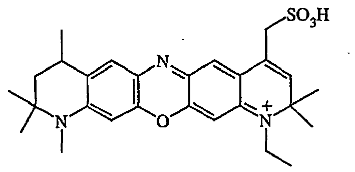 | | |
| **45** | nicht erfindunsgsgemäß | 673 | 698 |
| JA 324-S | 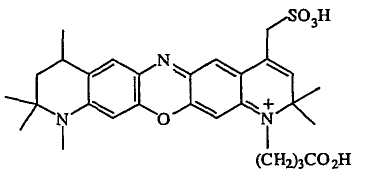 | | |
| **46** | nicht erfindunsgsgemäß | 655 | 679 |
| JA 326 | 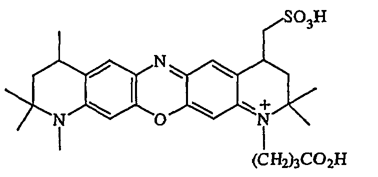 | | |
| **47** | nicht erfindunsgsgemäß | 654 | 678 |
| JA 329 | 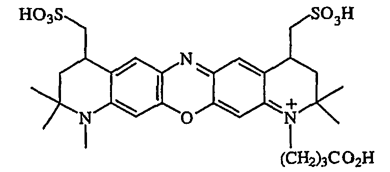 | | |
| **48** | nicht erfindunsgsgemäß | 694 | 717 |
| JA 410 | 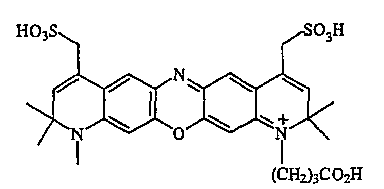 | | |
| **49** | nicht erfindunsgsgemäß | 674 | 695 |
| JA 331 | 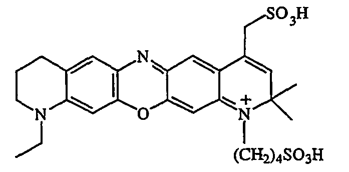 | | |
| **50** | nicht erfindunsgsgemäß | 654 | 678 |
| JA 366 | 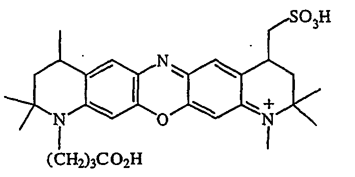 | | |
| **51** | nicht erfindunsgsgemäß | 653 | 676 |
| JA 403 | 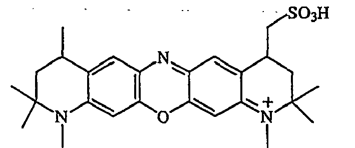 | | |
| **52** | nicht erfindunsgsgemäß | 678 | 699 |
| JA 404 | 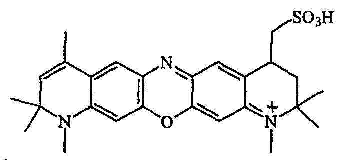 | | |
| **53** | nicht erfindunsgsgemäß | 694 | 715 |
| JA 408 | 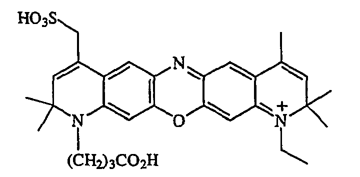 | | |

**Tabelle 2d:**

| | | | |
|---|---|---|---|
| **Carbopyroninfarbstoffe mit Sulfonsäure-Gruppe** | | | |
| Spektrale Daten in Ethanol: | λₐ Absorptionsmaximum, | | |
| | λ_{f} Fluoreszenzmaximum | | |

| Name | Farbstoff (Anion A⁻) | λₐ/nm | λ_{f}/nm |
|---|---|---|---|
| **54** | nicht erfindunsgsgemäß | 637 | 664 |
| JA 323 | 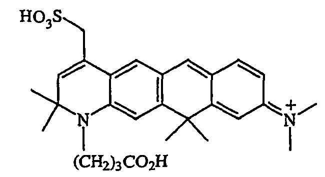 | | |
| **55** | nicht erfindunsgsgemäß | 648 | 675 |
| AZ 30 | 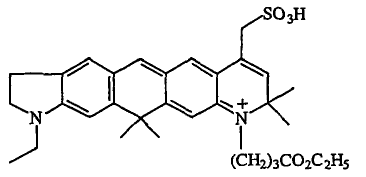 | | |
| **56** | nicht erfindunsgsgemäß | 648 | 674 |
| AZ 31 | 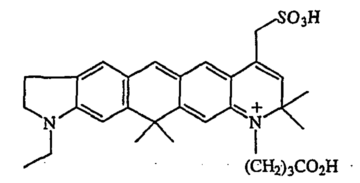 | | |
| **57** 35 | nicht erfindunsgsgemäß | 649 | 674 |
| AZ | 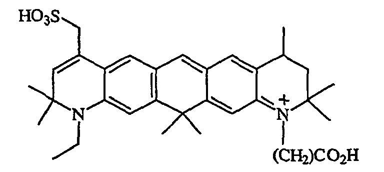 | | |
| **58** | nicht erfindunsgsgemäß | 641 | 666 |
| AZ 8-SO₃H | 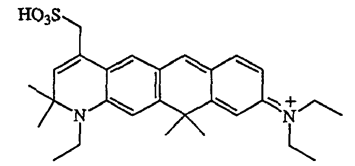 | | |
| **59** | nicht erfindunsgsgemäß | 647 | 675 |
| AZ 9-SO₃H | 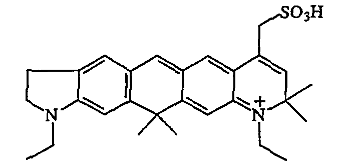 | | |
| **60** | nicht erfindunsgsgemäß | 637 | 664 |
| AZ 10-SO₃H | 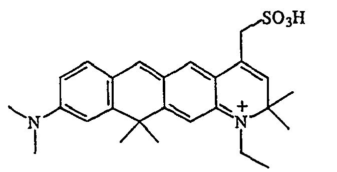 | | |
| **61** | nicht erfindunsgsgemäß | 664 | 688 |
| AZ 11-SO₃H | 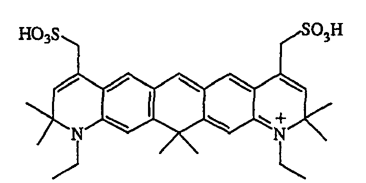 | | |
| **62** | nicht erfindunsgsgemäß | 647 | 674 |
| AZ 12-SO₃H | 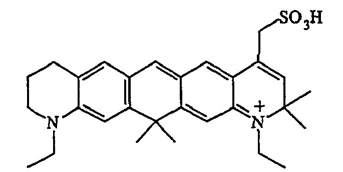 | | |

**Tabelle 2e:**

| | | | |
|---|---|---|---|
| **Amidopyryliumfarbstoffe mit Sulfonsäure-Gruppe** | | | |
| Spektrale Daten in Ethanol: | λₐ Absorptionsmaximum, . | | |
| | λ_{f} Fluoreszenzmaximum | | |

| Name | Struktur (Anion A⁻) | λₐ/nm | λ_{f}/nm |
|---|---|---|---|
| **63** | nicht erfindunsgsgemäß | 635 | 695 |
| NK 32 | 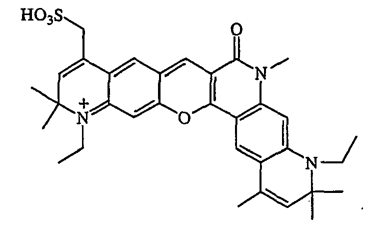 | | |
| **64** | nicht erfindunsgsgemäß | 610 | 668 |
| NK 34 | 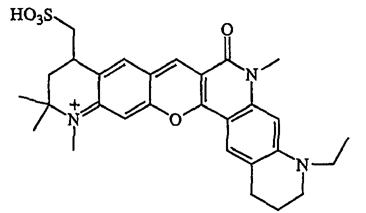 | | |

**Tabelle 3a:**

| | | | |
|---|---|---|---|
| **Sulfonsäurederivate erfindungsgemäßer Cumarinfarbstoffe** | | | |
| Spektrale Daten in Ethanol: | λₐ Absorptionsmaximum, | | |
| | λ_{f} Fluoreszenzmaximum | | |

| Name | Struktur | λₐ/nm | λ_{f}/nm |
|---|---|---|---|
| **65** | 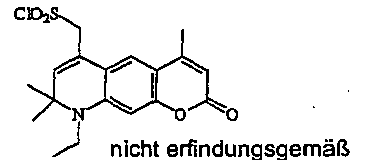 | 394 | 468 |
| AZ 96 | | | |
| **66** | 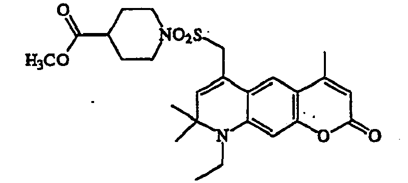 | 393 | 468 |
| AZ 97 | | | |
| **67** | 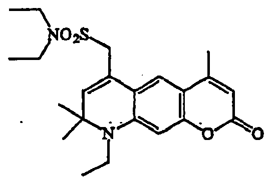 | 394 | 467 |
| AZ 98 | | | |
| **68** | 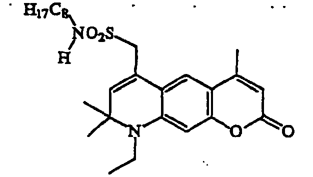 | 393 | 469 |
| AZ 99 | | | |

**Tabelle 3b:**

| | | | |
|---|---|---|---|
| **Sulfonsäurederivate erfindungsgemäßer Rhodaminfarbstoffe** | | | |
| Spektrale Daten in Ethanol: | λₐ Absorptionsmaximum, | | |
| | λ_{f} Fluoreszenzmaximum | | |

| Name | Struktur (Anion A⁻) | λₐ/nm | λ_{f}/nm |
|---|---|---|---|
| **69** | 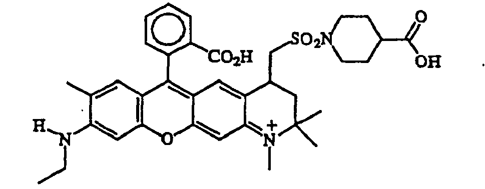 | 542 | 567 |
| AZ 49 | | | |
| **70** | 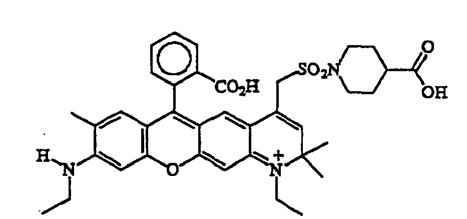 | 561 | 587 |
| AZ 50 | | | |
| **71** | 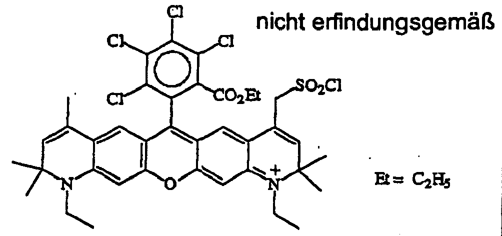 | 624 | 644 |
| AZ 84 | | | |
| **72** | 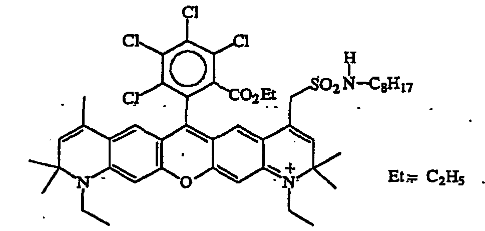 | 624 | 644 |
| AZ 85 | | | |
| **73** | 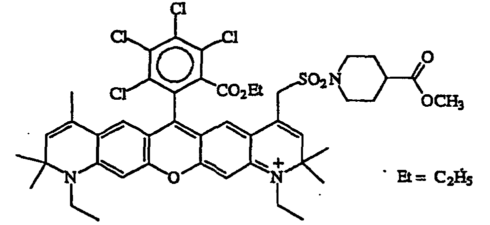 | 624 | 645 |
| AZ 86 | | | |
| **74** | 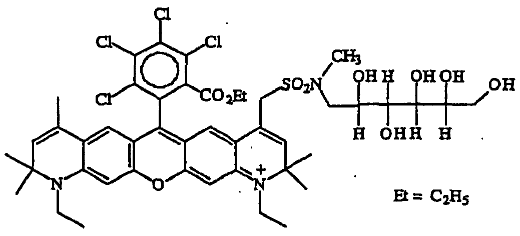 | 623 | 644 |
| AZ 88 | | | |
| **75** | 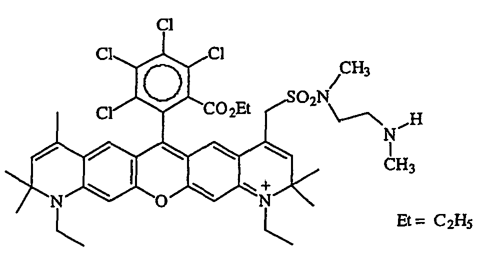 | 624 | 644 |
| AZ 89 | | | |
| **76** | 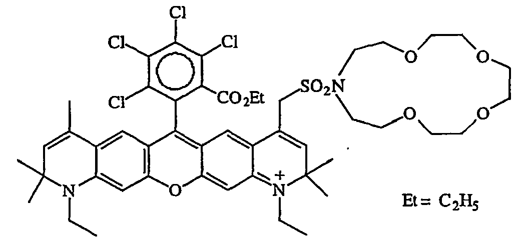 | 624 | 645 |
| AZ 90 | | | |
| **77** | 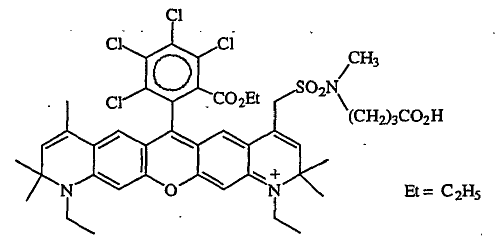 | 624 | 644 |
| AZ 87 | | | |
| **78** | 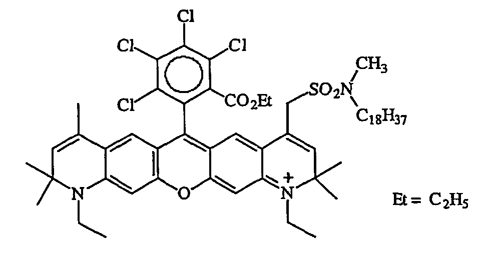 | 624 | 644 |
| AZ 93 | | | |
| **79** | 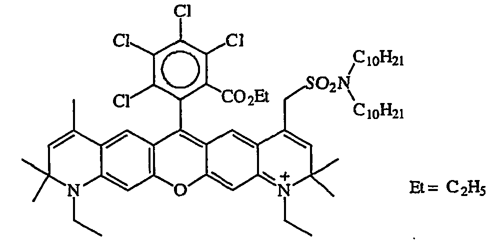 | 624 | 646 |
| AZ 94 | | | |
| **80** | 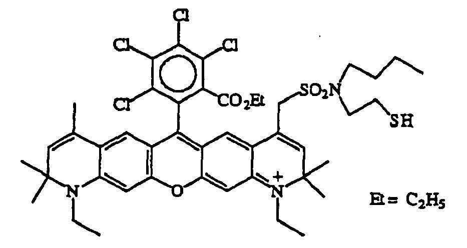 | 624 | 644 |
| AZ 95 | | | |
| **81** | 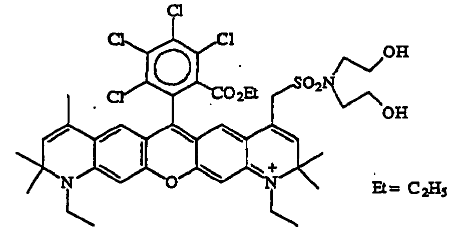 | 624 | 646 |
| AZ 91 | | | |
| **82** | 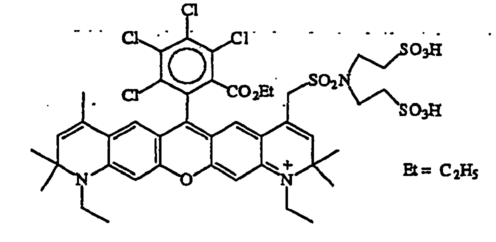 | 624 | 644 |
| AZ 92 | | | |

**Tabelle 3c:**

| | | | |
|---|---|---|---|
| **Sulfonsäurederivate erfindungsgemäßer Oxazinfarbstoffe** | | | |
| Spektrale Daten in Ethanol: | λₐ Absorptionsmaximum, | | |
| | λ_{f} Fluoreszenzmaximum | | |

| Name | Struktur (Anion A⁻) | λₐ/nm | λ_{f}/nm |
|---|---|---|---|
| **83** | 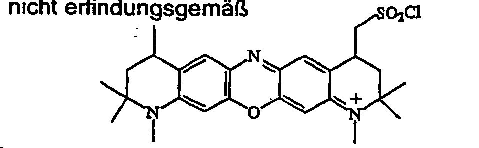 | 654 | 680 |
| AZ 46 | | | |
| **84** | 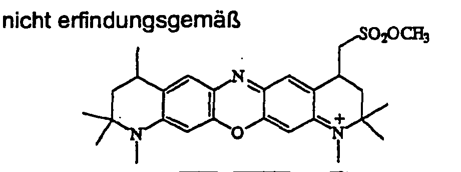 | 654 | 679 |
| JA 403 ME | | | |
| **85** | 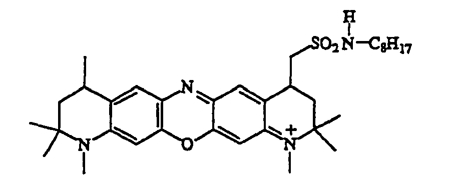 | 653 | 677 |
| AZ 54 | | | |
| **86** | 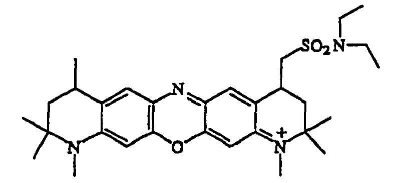 | 654 | 679 |
| AZ 55 | | | |
| **87** | 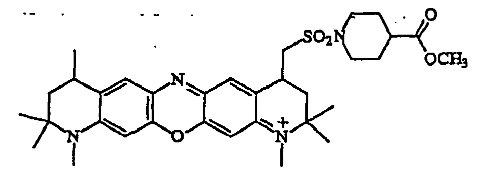 | 653 | 678 |
| AZ 56 | | | |
| **88** | 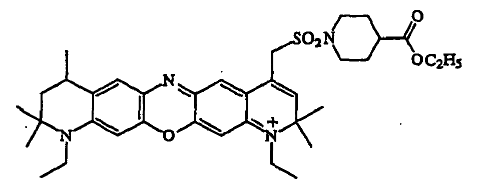 | 678 | 699 |
| AZ 52 | | | |
| **89** | 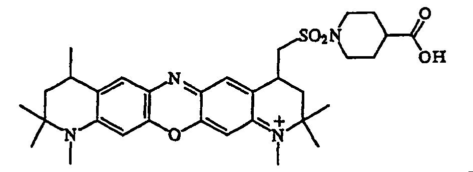 | 653 | 677 |
| AZ 57 | | | |
| **90** | 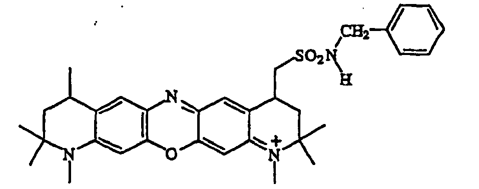 | 654 | 678 |
| AZ 102 | | | |
| **91** | 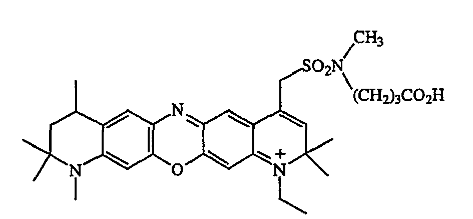 | 678 | 717 |
| AZ 73 | | | |
| **92** | 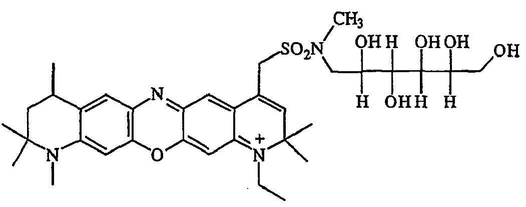 | 678 | 715 |
| AZ 74 | | | |
| **93** | 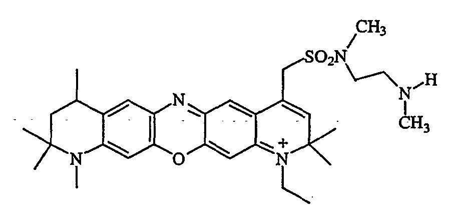 | 679 | 715 |
| AZ 75 | | | |
| **94** | 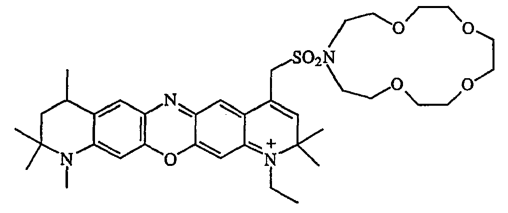 | 678 | 715 |
| AZ 76 | | | |
| **95** | 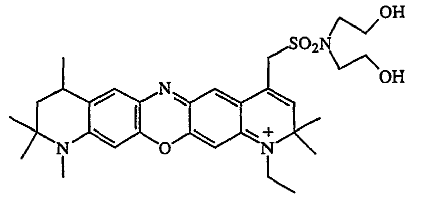 | 678 | 716 |
| AZ 77 | | | |
| **96** | 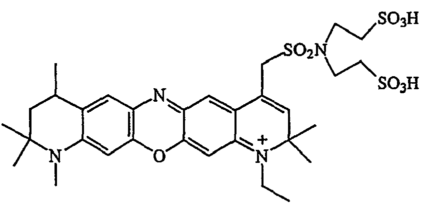 | 677 | 714 |
| AZ 78 | | | |
| **97** | 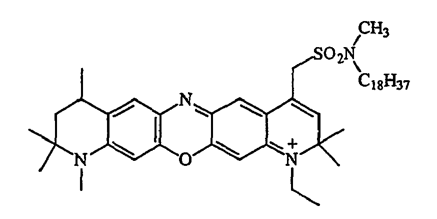 | 678 | 716 |
| AZ 79 | | | |
| **98** | 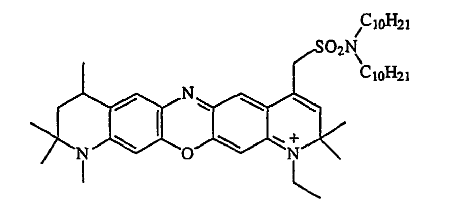 | 678 | 715 |
| AZ 80 | | | |
| **99** | 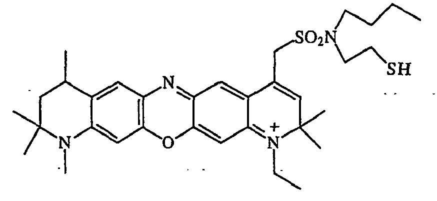 | 679 | 715 |
| AZ81 | | | |

**Tabelle 3d:**

| | | | |
|---|---|---|---|
| **Sulfonsäurederivate erfindungsgemäßer Carbopyroninfarbstoffe** | | | |
| Spektrale Daten in Ethanol: | λₐ Absorptionsmaximum, | | |
| | λ_{f} Fluoreszenzmaximum | | |

| Name | Struktur (Anion A⁻) | λₐ/nm λ | _{f}/nm |
|---|---|---|---|
| **100** | 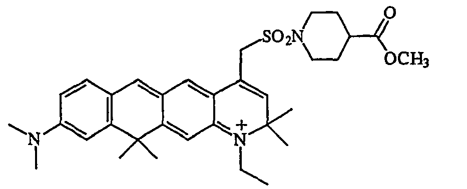 | 634 | 658 |
| AZ 48 | | | |
| **101** | 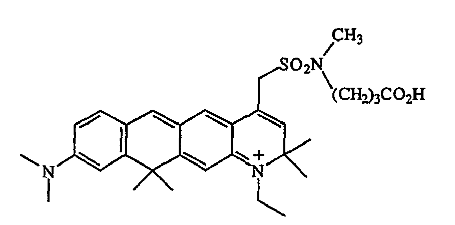 | 635 | 661 |
| AZ 64 | | | |
| **102** | 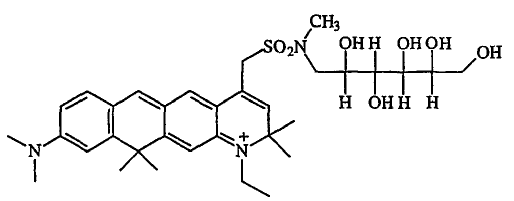 | 634 | 661 |
| AZ 65 | | | |
| **103** | 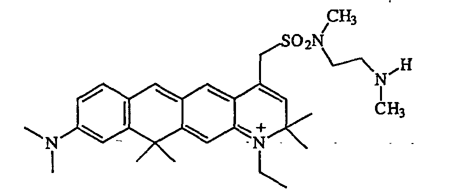 | 635 | 663 |
| AZ 66 | | | |
| **104** | 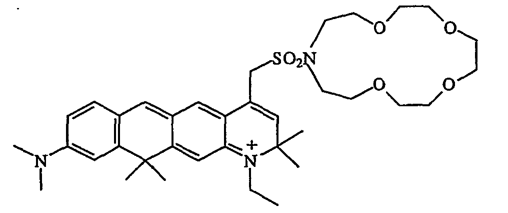 | 635 | 663 |
| AZ 67 | | | |
| **105** | 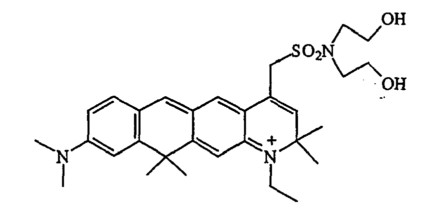 | 634 | 662 |
| AZ 68 | | | |
| **106** | 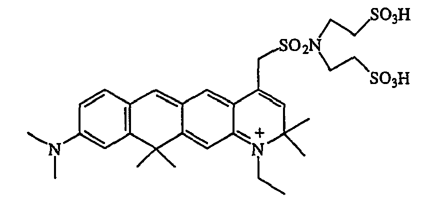 | 634 | 661 |
| AZ 69 | | | |
| **107** | 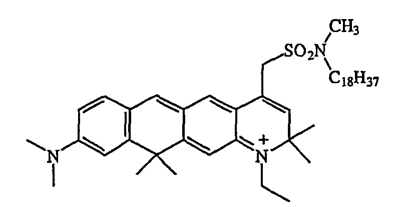 | 634 | 662 |
| AZ 70 | | | |
| **108** | 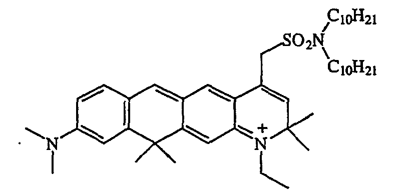 | 634 | 662 |
| AZ 71 | | | |
| **109** | 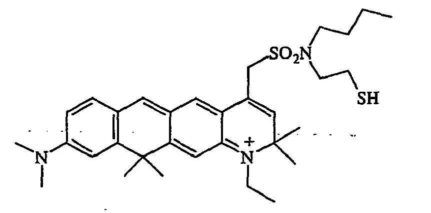 | 635 | 661 |
| AZ 72 | | | |
| **110** | 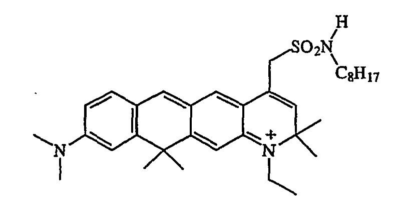 | 634 | 661 |
| AZ 82 | | | |
| **111** | 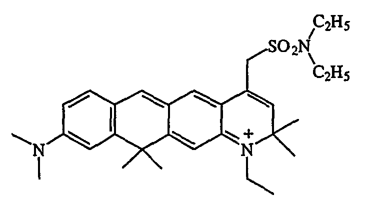 | 635 | 663 |
| AZ 83 | | | |

**Tabelle 3e:**

| | | | |
|---|---|---|---|
| **Sulfonsäurederivate erfindungsgemäßer Trifluormethyl-substituierter Xanthenfarbstoffe** | | | |
| Spektrale Daten in Ethanol: | λₐ Absorptionsmaximum, | | |
| | λ_{f} Fluoreszenzmaximum | | |

| Name | Struktur (Anion A⁻) | λₐ / nm | λ_{f} / nm |
|---|---|---|---|
| **112** | 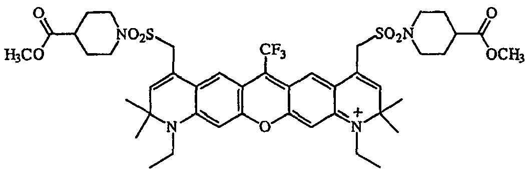 | 665 | 694 |
| AZ51 | | | |
| **113** | 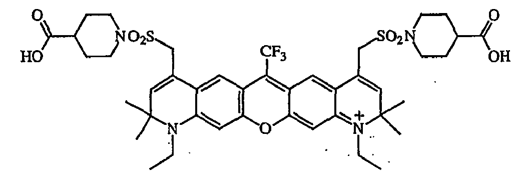 | 664 | 694 |
| AZ63 | | | |
| **114** | 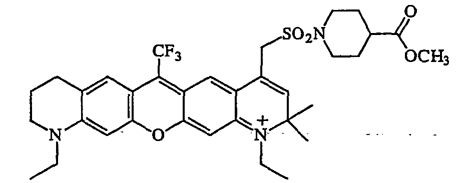 | 651 | 680 |
| AZ60 | | | |
| **115** | 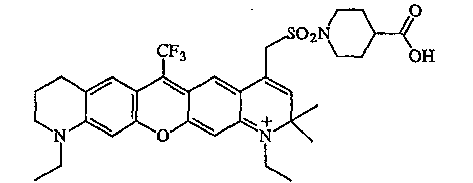 | 652 | 680 |
| AZ62 | | | |
| **116** | 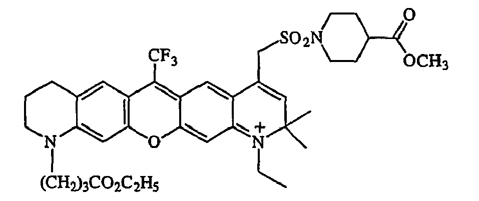 | 652 | 679 |
| AZ 61 | | | |

### Beispiele

Die Erfindung wird durch nachfolgende Beispiele näher erläutert. Es sind sowohl Beispiele für die Herstellung der sulfonierten Chinolinvorstufen und ihre Derivatisierung als auch Beispiele für die Synthese und Modifizierung von Farbstoffen aus den entsprechend sulfonierten Chinolinen angegeben. Die als Ausgangsverbindungen für die unter 1. beschriebenen Verbindungen verwendeten Dihydro- und Tetrahydrochinoline und primären bzw. sekundären Amine sind entweder kommerziell erhältlich oder nach literaturbekannten Synthesen oder dem Fachmann bekannten Verfahren darzustellen. Dies trifft auch für die Ausgangsfarbstoffe der unter 2. beschriebenen Farbstoffderivate zu.

### 1. Vorstufen: Herstellung erfindungsgemäßer Verbindungen

### Verbindung 8 (nicht erfindungsgemäß)

Zur Darstellung von (1-Ethyl-2,2-dimethyl-1,2-dihydrochinol-4-yl)-methansulfonsäure (8) werden 6,0 g (21 mmol) 1-Ethyl-2,2,4-dimethyl-1,2-dihydrochinolin unter Eiskühlung in einem Gemisch aus 10 ml konzentrierter Schwefelsäure und Oleum im Verhältnis 5:1 gelöst. Es wird 20 Stunden bei Raumtemperatur verrührt. Das Reaktionsgemisch wird auf Eis gegossen und mit 20 %iger Natronlauge unter Eis/Methanol-Kühlung alkalisch gestellt (pH12). Zur Entfernung des nicht umgesetzten Edukts wird mit Chloroform extrahiert und die wässrige Phase bis zur Trockene am Rotationsverdampfer eingeengt. Der kristalline Rückstand wird in Ethanol suspendiert, vom Natriumsulfat abgesaugt und die organische Phase am Rotationsverdampfer zur Trockene eingeengt. Der harzige Rückstand kristallisiert bei Zugabe von Aceton. Es wird erneut abgesaugt, mit Aceton gewaschen und unter Vakuum über Phosphorpentoxid getrocknet. Das erhaltene Rohprodukt kann ohne weitere Reinigungsschritte in der nächsten Stufe eingesetzt werden. Ausbeute 82 %.

ESI-Massenspektrum: m/z = 282 (MH⁺)
NMR (DMSO-d₆): CH₃CH₂-, 0.97ppm, T, 3; -CH₂-N, 3.39ppm, Q, 2; 2x CH₃-, 1.31ppm, S, 6; =CH, 5.74ppm, S, 1; -CH₂SC₃H, 4.27ppm, S, 2; H aromat, 7.15-7,58ppm, M, 4.

### Verbindung 9 (nicht erfindungsgemäß)

Zur Darstellung von (1-Ethyl-2,2-dimethyl-1,2-dihydrochinol-4-yl)-methansulfonsäure-chlorid **(9)** werden 2,5 g (8,8 mmol) (1-Ethyl-2,2-dimethyl-1,2-dihydrochinol-4-yl)-methansulfonsäure **(8)** in 70 ml trockenem Benzol suspendiert und bei Raumtemperatur portionsweise mit Phosphorpentachlorid 4,6 g (22,2 mmol) versetzt. Die Reaktionsmischung färbt sich zunächst gelb, später gelborange. Der weiße Niederschlag wird nach 1.5 Stunden abfiltriert und mit kaltem trockenem Benzol gewaschen. Es wird im Vakuum über Phosphorpentoxid getrocknet. Das erhaltene Säurechlorid wird ohne weitere Reinigung sofort in der nächsten Stufe eingesetzt.
Ausbeute 75 %.
ESI-Massenspektrum: m/z = 300 (MH⁺)

### Verbindung 10

Zur Darstellung von (1-Ethyl-2,2-dimethyl-1,2-dihydrochinol-4-yl-methansulfonyl)-piperidin-4-carbonsäuremethylester **(10)** werden 2 g (6,6 mmol) (1-Ethyl-2,2-dimethyl-1,2-dihydrochinol-4-yl)-methansulfonsäurechlorid **(9)** in 40 ml trockenem Acetonitril gelöst und mit einem Eisbad gekühlt. Man versetzt tropfenweise mit 1,13 g (7,9 mmol) 4-Piperidincarbonsäuremethylester gefolgt von 0,85 g (6,6 mmol) Diisopropylethylamin. Die Reaktionsmischung wird 30 Minuten bei Raumtemperatur verrührt. Man versetzt mit Wasser und extrahiert mit Chloroform, wäscht mit 10 %iger kalter Sodalösung und trocknet über wasserfreiem Natriumsulfat. Das Lösungsmittel wird am Rotationsverdampfer abdestilliert und der Rückstand an Kieselgel mit einem von Chloroform nach Ethanol verlaufenden Gradienten säulenchromatographisch gereinigt. Ausbeute 82 %.
ESI-Massenspektrum: m/z = 407 (MH⁺)

### Verbindung 13

Zur Darstellung von 1-(1-Ethyl-6-formyl-2,2-dimethyl-1,2-dihydrochinol-4-yl-methan-sulfonyl)-piperidin-4-carbonsäuremethylester **(13)** werden 3 ml trockenes Dimethylformamid im Eis/Methanol Bad auf -10 °C gekühlt und tropfenweise mit 0,45 ml (4,9 mmol) Phosphoroxidtrichlorid versetzt. Die Reaktionsmischung wird 20 Minuten bei -5 °C verrührt. (1-Ethyl-2,2-dimethyl-1,2-dihydrochinol-4-yl-methansulfonyl)-piperidin-4-carbonsäure-methylester **(10)** werden in 1,5 ml trockenem Dimethylformamid gelöst und bei -5 °C dem Reaktionsgemisch zugetropft. Nach beendeter Zugabe wird 50 Minuten auf 80 °C erhitzt. Die Reaktionslösung wird auf Eiswasser gegossen und mit Chloroform versetzt. Es wird mit 20 %iger Natronlauge auf pH12 eingestellt und mehrmals mit Chloroform extrahiert. Die organische Phase wird mit 10 % iger Sodalösung gewaschen und über wasserfreiem Natriumsulfat getrocknet. Das erhaltene Rohprodukt wird am Rotationsverdampfer unter Vakuum zur Trockene eingeengt und an Kieselgel über einem von Chloroform nach Ethanol verlaufenden Gradienten säulenchromatographisch gereinigt.
Ausbeute 88 %.
ESI-Massenspektrum: m/z = 435 (MH⁺)
NMR (CDCl₃): C**H**₃CH₂-, 1.22ppm, T, 3; -C**H**₂-N, 3.41ppm, Q, 2; 2x C**H**₃-, 1.42ppm, S, 6; =C**H**, 5.63ppm, S, 1; -C**H**₂SO₂N, 4.00ppm, S, 2; **H**-5 aromat, 7.59ppm, S, 1; **H**-7 aromat, 7.57ppm, D, 1; **H**-8 aromat, 6.55ppm, D, 1; **H-**C=O, 9.68ppm, S, 1; -O-C**H**₃, 3.63ppm, S, 1; C**H**(Piperidin)-C=O, 2.31 ppm, M, 1; N-C**HH**'(Piperidin), 2.83ppm u. 3.52ppm; M, 2,2; HC-C**HH**'(Piperidin), 1.67ppm u. 1.83ppm; M, 2,2.

### Verbindung 14

Zur Darstellung von 1-(1-Ethyl-6-hydroxymethyl-2,2-dimethyl-1,2-dihydrochinol-4-yl-methansulfonyl)-piperidin-4-carbonsäuremethylester **(14)** werden 1 g (2,3 mmol) 1-(1-Ethyl-6-formyl-2,2-dimethyl-1,2-dihydrochinol-4-yl-methansulfonyl)-piperidin-4-carbonsäuremethylester **(13)** in 10 ml Ethanol gelöst und unter Eiskühlung mit 0,05g Natriumborhydrid versetzt, wobei eine deutliche Gasentwicklung mit Schäumen zu beobachten ist. Es wird 50 Minuten bei Raumtemperatur verrührt. Zur Zersetzung des Überschusses an Reduktionsmittel wird tropfenweise mit 1 N Salzsäure versetzt bis kein Schäumen mehr zu beobachten ist (pH7). Man gießt auf 50 ml Wasser und extrahiert 3 mal mit 20 ml Chloroform. Die vereinigten organischen Phasen werden über wasserfreiem Natriumsulfat getrocknet und das Lösungsmittel am Rotationsverdampfer bis zur Trockene abdestilliert. Der erhaltene Rückstand wird an Kieselgel mit einem von Chloroform nach Ethanol verlaufenden Gradienten säulenchromatographisch gereinigt.
Ausbeute 88 %.
ESI-Massenspektrum: m/z = 435 (MH⁺)
NMR (CDCl₃): C**H**₃CH₂-, 1.10ppm, T, 3; -C**H**₂-N, 3.25ppm, Q, 2; 2x C**H**₃-, 1.27ppm, S, 6; =C**H**, 5.53ppm, S, 1; -C**H**₂SO₂N, 3.96ppm, S, 2; **H**-5 aromaten, 7.10ppm, S, 1; **H**-7 aromaten, 7.05ppm, D, 1; **H**-8 aromaten, 6.46ppm, D, 1; **H₂**C-OH, 4.47ppm, S, 1; -O-C**H₃**, 3.57ppm, S, 1; C**H**(Piperidin)-C=O, 2.29ppm, M, 1; N-C**HH**'(Piperidin), 2.82ppm u. 3.53ppm; M, 2,2; HC-C**HH'**(Piperidin), 1.68ppm u. 1.73ppm; M, 2,2; -CH₂-O**H**, nn.

### Verbindung 3 (nicht erfindungsgemäß)

Zur Darstellung von (1-Methyl-7-methoxy-2,2-dimethyl-1,2,3,4-tetrahydrochinol-4-yl)-methansulfonsäure **(3)** werden 5,0 g (1-Methyl-7-methoxy-2,2-dimethyl-1,2-dihydrochinol-4-yl)-methansulfonsäure werden in 100 ml Methanol gelöst und mit 0,5 g 10 %igem Palladium auf Aktivkohle versetzt. Das Reaktionsgemisch wird im Autoklaven 18 Stunden bei 70 bar und Raumtemperatur hydriert. Man filtriert und engt am Rotationsverdampfer zur Trockene ein. Der erhaltene Feststoff lässt sich ohne weitere Reinigung in der nächsten Stufe einsetzen.
Ausbeute 90 %.
ESI-Massenspektrum: m/z = 270 (MH⁺)

### Verbindung 11

Die Darstellung von N,N-Diethyl-(1-Ethyl-2,2-dimethyl-1,2-dihydrochinol-4-yl)-methansulfonamid **(11)** erfolgt analog Verbindung **(10)** unter Verwendung von Verbindung **(9)** mit Diethylamin. Die säulenchromatographische Isolierung des Produktes erfolgt an Kieselgel mit einem von Chloroform nach Ethanol verlaufenden Gradienten.
Ausbeute 79 %.
ESI-Massenspektrum: m/z = 337 (MH⁺)

### Verbindung 12

Die Darstellung von (1-Ethyl-2,2-dimethyl-1,2-dihydrochinol-4-yl)-N-octylmethansulfonamid **(12)** erfolgt analog Verbindung **(10)** unter Verwendung von Verbindung **(9)** mit n-Octylamin. Die säulenchromatographische Isolierung des Produktes erfolgt an Kieselgel mit einem von Chloroform nach Ethanol verlaufenden Gradienten.
Ausbeute 81 %
ESI-Massenspektrum: m/z = 393 (MH⁺)

### Verbindung 15 (nicht erfindungsgemäß)

Zur Darstellung von (1-Ethyl-7-methoxy-2,2-dimethyl-1,2-dihydrochinol-4-yl)-methansulfonsäure **(15)** werden 20 g (8,6 mmol) 1-Ethyl-7-methoxy-2,2,4-dimethyl-1,2-dihydrochinolin unter Eiskühlung in einem Gemisch aus 10 ml konzentrierter Schwefelsäure und Oleum im Verhältnis 5:1 gelöst. Es wird 20 Stunden bei Raumtemperatur verrührt. Das Reaktionsgemisch wird auf Eis gegossen und mit 20 %iger Natronlauge unter Eis/Methanol Kühlung alkalisch gestellt (pH12). Zur Entfernung des nicht umgesetzten Edukts wird mit Chloroform extrahiert und die wässrige Phase zur Trockene am Rotationsverdampfer eingeengt. Der kristalline Rückstand wird säulenchromatographisch an Kieselgel mit einem von Chloroform nach Ethanol verlaufenden Gradienten gereinigt.
Ausbeute 86 %.
ESI-Massenspektrum: m/z = 312 (MH⁺)
NMR (DMSO-d₆): C**H₃**CH₂-, 1.09ppm, T, 3; -C**H₂**-N, 3.25ppm, Q, 2; 2x C**H₃**-, 1.24ppm, S, 6; =C**H**, 5.28ppm, S, 1; -C**H₂**SO₃H, 3.43ppm, S, 2; **H**-5 aromat, 6.04ppm, D, 1; **H**-6 aromat, 7.17ppm, D, 1; **H-**8 aromat, 5.93ppm, S, 1; CH₃O-, 3.68ppm, S, 3.

### Verbindung 16 (nicht erfindungsgemäß)

Die Darstellung von (1-Ethyl-7-methoxy-2,2-dimethyl-1,2-dihydrochinol-4-yl)-methan-sulfonsäurechlorid **(16)** erfolgt analog Verbindung **(9)** aus Verbindung **(15)** und Phosphorpentachlorid in trockenem Benzol. Das erhaltene Säurechlorid wird sofort zur weiteren Umsetzung eingesetzt.
Ausbeute 73 %.
ESI-Massenspektrum: m/z = 330 (MH⁺)

### Verbindung 17

Die Darstellung von (1-Ethyl-7-methoxy-2,2-dimethyl-1,2-dihydrochinol-4-yl-methansulfonyl)-piperidin-4-carbonsäuremethylester **(17)** erfolgt analog Verbindung **(10)** aus Verbindung **(16)** in trockenem Acetonitril bei Raumtemperatur.
Ausbeute 69 %.
ESI-Massenspektrum: m/z = 437 (MH⁺)
NMR (CDCl₃): C**H₃**CH₂-, 1.15ppm, T, 3; -C**H₂**-N, 3.27ppm, Q, 2; 2x C**H₃**-, 1.31ppm, S, 6; =C**H**, 5.41 ppm, S, 1; -C**H₂**SO₂N, 3.97ppm, S, 2; **H**-5 aromat, 6.17ppm, D, 1; **H**-6 aromat, 7.07ppm, D, 1; **H**-8 aromat, 6.09ppm, S, 1; -O-C**H₃**, 3.77ppm, S, 1; CH(Piperidin)-C=O, 2.31ppm, M, 1; N-C**HH'**(Piperidin), 2.82ppm u. 3.63ppm; M, 2,2; HC-C**HH'**(Piperidin), 1.63ppm u. 1.80ppm; M, 2,2; O=C-OC**H₃**, 3.63, S, 3.

### Verbindung 18 (nicht erfindungsgemäß)

Zur Darstellung von (1-Ethyl-7-hydroxy-2,2-dimethyl-1,2-dihydrochinol-4-yl)-methan-sulfonsäure **(18)** werden 5 g (16,1 mmol) (1-Ethyl-7-methoxy-2,2-dimethyl-1,2-dihydrochinol-4-yl)-methansulfonsäure **(16)** in 10 ml 48 %iger Bromwasserstoffsäure suspendiert und 2 Stunden zum Rückfluss erhitzt. Man gießt auf Eis, versetzt mit Chloroform und neutralisiert mit 20 %iger Natronlauge. Die organische Phase wird abgetrennt und die wässrige Phase am Rotationsverdampfer im Vakuum zur Trockene einrotiert. Der Rückstand wird in heißem Ethanol suspendiert, filtriert und das Filtrat erneut bis zur Trockene einrotiert. Das so erhaltene Produkt wird ohne weitere Reinigung in der nächsten Stufe eingesetzt.
Ausbeute 91 %.
ESI-Massenspektrum: m/z = 298 (MH⁺)

### Verbindung 19 (nicht erfindungsgemäß)

Die Darstellung von (1-Methyl-7-methoxy-2,2-dimethyl-1,2,3,4-tetrahydrochinol-4-yl)-methansulfonsäurechlorid **(19)** erfolgt analog Verbindung **(16)** unter Verwendung von Verbindung **(3)** und Phosphorpentachlorid mit dem Unterschied, dass 18 Stunden bei Raumtemperatur verrührt wird.
Ausbeute 78 %.
ESI-Massenspektrum: m/z = 317 (MH⁺)

### Verbindung 20

Die Darstellung von (1-Methyl-7-methoxy-2,2-dimethyl-1,2,3,4-tetrahydrochinol-4-yl-methansulfonyl)-piperidin-4-carbonsäuremethylester **(20)** erfolgt analog Verbindung **(10)** aus Verbindung **(19)** mit 4-Piperidincarbonsäuremethylester in trockenem Acetonitril. Die säulenchromatographische Isolierung erfolgt an Kieselgel mit einem von Chloroform nach Ethanol verlaufenden Gradienten.
Ausbeute 69 %.
ESI-Massenspektrum: m/z = 425 (MH⁺)

### Verbindung 21

Die Darstellung von (1-Methyl-7-methoxy-2,2-dimethyl-1,2,3,4-tetrahydrochinol-4-yl)-N-octyl-methansulfonamid **(21)** erfolgt analog Verbindung **(10)** aus Verbindung **(19)** mit n-Octylamin in trockenem Acetonitril. Die säulenchromatographische Isolierung erfolgt an Kieselgel mit einem von Chloroform nach Ethanol verlaufenden Gradienten.
Ausbeute 69 %.
ESI-Massenspektrum: m/z = 411 (MH⁺)

### 2. Farbstoffe: Herstellung erfindungsgemäßer Verbindungen

### Verbindung 55 (AZ 30) (nicht erfindungsgemäß)

1,2 g (4,93 mmol) 4-(6-Hydroxymethyl-2,2,4-trimethyl-2H-chinol-1-yl)-buttersäure-ethylester werden zusammen mit 0,72 g (4,93 mmol) 1-Ethyl-6-isopropenyl-indolin in 20 ml trockenem Dichlormethan gelöst und auf -5 °C gekühlt. Man versetzt tropfenweise mit 7 ml einer 1 molaren Lösung von Bortrichlorid in Dichlormethan. Das Reaktionsgemisch wird 2 Stunden bei Raumtemperatur verrührt, das Lösungsmittel am Rotationsverdampfer entfernt und der Rückstand in konzentrierter Schwefelsäure gelöst. Die Reaktionsmischung wird 4 Stunden bei Raumtemperatur verrührt. Man tropft in eiskaltes Ethanol, versetzt mit 0,8 g Tetrabutylammoniummetaperiodat und erhitzt kurz zum Sieden. Man lässt abkühlen, versetzt mit Wasser und nimmt den Farbstoff in Chloroform auf. Es wird über wasserfreiem Natriumsulfat getrocknet und die Farbstofflösung am Rotationsverdampfer zur Trockene eingeengt. Das erhaltene Farbstoffrohprodukt wird an Kieselgel mit einem von Chloroform nach Ethanol verlaufenden Gradienten säulenchromatographisch gereinigt.
Ausbeute 45 %.
ESI-Massenspektrum: m/z = 565 (M⁺)
Absorptionsmaximum: λₐ = 648 nm (in Ethanol)

### Verbindung 83 (AZ 46) (nicht erfindungsgemäß)

100 mg (0,21 mmol) JA 403 werden in 8 ml trockenem Acetonitril gelöst und im kalten Wasserbad gekühlt. Man versetzt mit 4 Tropfen trockenem Dimethylformamid gefolgt von 6 Tropfen frisch destilliertem Thionylchlorid. Es wird 45 Minuten verrührt. Die Umsetzung erfolgt quantitativ. Das Reaktionsgemisch wird in einem Eisbad gekühlt. Die Farbstofflösung wird in eine mit Eis/Methanol gekühlte 20 %ige Natriumperchloratlösung getropft. Der sich in feinen Kristallen abscheidende Farbstoff wird abgesaugt, mit wenig kaltem Wasser gewaschen und im Ölpumpenvakuum über Phosphorpentoxid getrocknet. Der Farbstoff kann ohne zusätzliche Reinigung für weitere Umsetzungen eingesetzt werden.
Ausbeute 81 %.
ESI-Massenspektrum: m/z = 502 (M⁺)
Absorptionsmaximum: λₐ = 650 nm (Acetonitril)

### Verbindung 90 (AZ 102)

10 mg (0.017 mmol) AZ 46 werden in 5 ml trockenem Acetonitril gelöst und mit 5 ml einer 10 mmolaren Lösung aus Benzylamin in trockenem Acetonitril versetzt. Das Reaktionsgemisch wird 20 Minuten bei Raumtemperatur verrührt. Die Reaktionslösung wird zur Trockene einrotiert und an Kieselgel mit einem von Chloroform nach Ethanol verlaufenden Gradienten säulenchromatographisch gereinigt.
Ausbeute 88 %.
ESI-Massenspektrum: m/z = 573 (M⁺)
Absorptionsmaximum: λₐ = 653 nm (in Ethanol)

### Verbindung 87 (AZ 56)

Die Synthese erfolgt analog zur Darstellung von AZ 102 unter Verwendung einer 10 mmolaren Lösung von 4-Piperidincarbonsäuremethylester. Die Isolierung erfolgt säulenchromatographisch an Kieselgel mit einem von Chloroform nach Ethanol verlaufenden Gradienten.
Ausbeute 91 %.
ESI-Massenspektrum: m/z = 609 (M⁺)
Absorptionsmaximum: λₐ = 652 (in Ethanol)

### Verbindung 89 (AZ 57)

10 mg (0,014 mmol) AZ 56 werden in 20 ml Acetonitril Wasser 1:1 gelöst und mit 0,5 ml 2 N Salzsäure versetzt. Die Reaktionsmischung wird 7 Stunden zum Rückfluss erhitzt. Die Farbstofflösung wird unter Vakuum eingeengt und an Kieselgel mit einem von Chloroform nach Ethanol verlaufenden Gradienten säulenchromatographisch gereinigt.
Ausbeute 62 %.
ESI-Massenspektrum: m/z = 595 (M⁺)
Absorptionsmaximum: λₐ = 652 nm (in Ethanol)

### Verbindung 34 (AZ 59) (nicht erfindungsgemäß)

500 mg (1,7 mmol) (1-Ethyl-7-hydroxy-2,2-dimethyl-1,2-dihydroChinol-4-yl)-methan-sulfonsäure (18), 440 mg (3,4 mmol) Acetessigsäureethylester und 460 mg trockenes Zinkchlorid werden in 40 ml absolutem Ethanol suspendiert und 24 Stunden zum Rückfluss erhitzt. Die Reaktionsmischung wird filtriert, am Rotationsverdampfer bis zur Trockene eingeengt und der Rückstand an Kieselgel mit einem von Chloroform nach Ethanol verlaufenden Gradienten säulenchromatographisch gereinigt.
Ausbeute 60 %.
ESI-Massenspektrum: m/z = 364 (MH⁺)
Absorptionsmaximum: λₐ = 394 nm (in Ethanol)

### Verbindung 71 (AZ 84) (nicht erfindungsgemäß)

Die Synthese erfolgt analog AZ 46 unter Verwendung von JA 407-E mit Thionylchlorid/DMF in trockenem Acetonitril. Der erhaltene Farbstoff wird als Perchlorat isoliert und getrocknet. Das Farbstoffsulfochlorid kann ohne weitere Reinigung sofort weiter umgesetzt werden.
Ausbeute 81 %.
ESI-Massenspektrum: m/z = 811 (M⁺)
Absorptionsmaximum: λₐ = 623 nm (in Ethanol)

### Verbindung 72 (AZ 85)

Die Darstellung erfolgt analog AZ 102 unter Verwendung von AZ 84 und n-Octylamin in trockenem Acetonitril. Die säulenchromatographische Isolierung erfolgt an Kieselgel mit einem von Chloroform nach Ethanol verlaufenden Gradienten.
Ausbeute 73 %.
ESI-Massenspektrum: m/z = 904 (M⁺)

### Verbindung 65 (AZ 96) (nicht erfindungsgemäß)

Die Darstellung erfolgt analog Verbindung (9) aus AZ 59 mit Phosphorpentachlorid in Benzol wobei 6 Stunden bei Raumtemperatur verrührt wird.
Ausbeute 63 %.
ESI-Massenspektrum: m/z = 383.2 (MH⁺)
Absorptionsmaximum: λₐ = 392 nm (in Ethanol)

### Verbindung 66 (AZ 97)

Die Darstellung erfolgt analog AZ 102 aus AZ 96 mit 4-Piperidincarbonsäuresäureethylester. Die säulenchromatographische Isolierung erfolgt an Kieselgel mit einem von Chloroform nach Ethanol verlaufenden Gradienten.
Ausbeute 76 %.
ESI-Massenspektrum: m/z = 489.2 (MH⁺)
Absorptionsmaximum: λₐ = 393 nm (in Ethanol)

### Verbindung 84 (JA 403 ME) (nicht erfindungsgemäß)

100 mg (0,17 mmol) JA 403 werden mit 0,5 ml Dimethylsulfat in 20 ml trockenem Acetonitril gelöst und für ca. 5 Stunden zum Rückfluss erhitzt. Die Reaktion wird dünnschicht-chromatographisch verfolgt. Nach Beendigung der Umsetzung wird die Reaktionsmischung zur Trockne eingeengt, in wenig Chloroform aufgenommen und der Rückstand an Kieselgel mit einem von Chloroform nach Ethanol verlaufenden Gradienten säulenchromatographisch gereinigt.
Ausbeute 70 %.
ESI-Massenspektrum: m/z = 498 (M⁺)
Absorptionsmaximum: λₐ = 654 nm (in Ethanol)

## Patentansprüche

1. Verfahren zur Herstellung von Dihydrochinolinverbindungen der allgemeinen Formel Ia oder von Tetrahydrochinolinverbindungen der allgemeinen Formel Ib 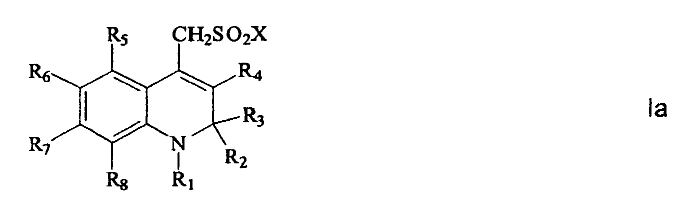 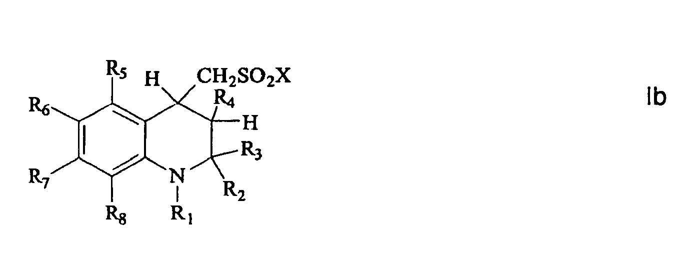 worin R₁ eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann,
R₂, R₃, R₄, R₅, R₆, R₇ und R₈ bei jedem Auftreten unabhängig voneinander Wasserstoff, Halogen, eine Hydroxy-, Amino-, Sulfo-, Carboxy- oder Aldehydgruppe oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, oder die Reste R₁ und R₈ zusammen ein Ringsystem bilden und
X -S-R₁₀ oder -NR₁₁R₁₂ bedeutet, wobei R_{10,} R₁₁ und R₁₂ unabhängig voneinander Wasserstoff oder einen C1 bis C20 Kohlenwasserstoffrest bedeuten, welcher gegebenenfalls ein oder mehrere Heteroatome oder/und einen oder mehrere Substituenten enthalten kann,
**dadurch gekennzeichnet,**
**dass** man die entsprechenden Verbindungen I'a 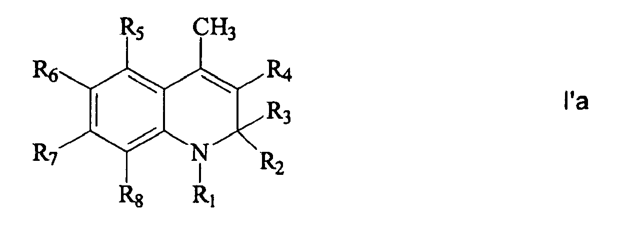 zu Ia (X = OH) sulfoniert und gegebenenfalls durch Hydrierung zu Ib (X = OH) umsetzt.

2. Verfahren nach Anspruch 1,
**dadurch gekennzeichnet,**
**dass** man die Sulfonierung mittels konzentrierter Schwefelsäure durchführt.

3. Verfahren nach Anspruch 1 oder 2,
**dadurch gekennzeichnet,**
**dass** man die bei der Sulfonierung gebildete Sulfonsäuregruppe derivatisiert.

4. Verfahren nach Anspruch 3,
**dadurch gekennzeichnet,**
**dass** man die Sulfonsäuregruppe zum Sulfochlorid umsetzt.

5. Verfahren nach Anspruch 3 oder 4,
**dadurch gekennzeichnet,**
**dass** man die Sulfochloridgruppe mit einem primären oder sekundären Amin zum Sulfonamid umsetzt.

6. Dihydrochinolinverbindung der allgemeinen Formel Ia oder Tetrahydrochinolinverbindung der allgemeinen Formel Ib 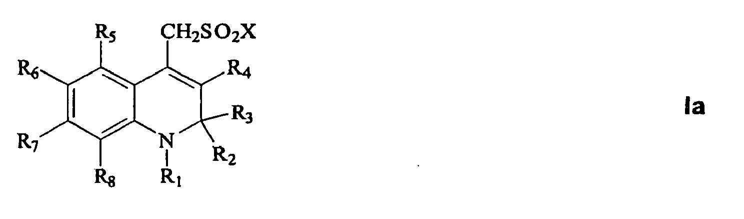 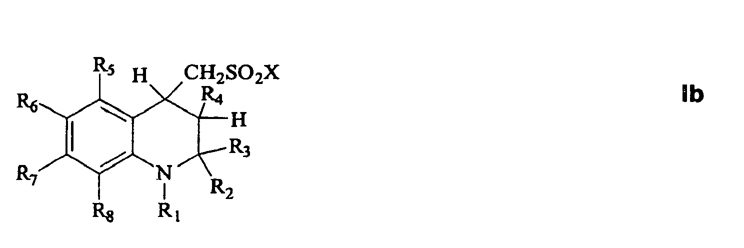 worin R₁ eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, R₂, R₃, R₄, R₅, R₆, R₇ und R₈ bei jedem Auftreten unabhängig voneinander Wasserstoff, Halogen, eine Hydroxy-, Amino-, Sulfo-, Carboxy- oder Aldehydgruppe oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, oder die Reste R₁ und R₈ zusammen ein Ringsystem bilden und
X -S-R₁₀ oder -NR₁₁R₁₂ bedeutet, wobei R₁₀, R₁₁ und R₁₂ jeweils unabhängig voneinander Wasserstoff oder einen C1 bis C20 Kohlenwasserstoffrest bedeuten, welcher gegebenenfalls ein oder mehrere Heteroatome oder/und einen oder mehrere Substituenten, insbesondere -SO₃H, -PO₃H₂ und -COOH enthalten kann.

7. Verbindung nach Anspruch 6,
worin R₁ einen Aryl- oder Alkylrest, insbesondere einen C5 bis C15-Aryloder einen C1 bis C20-Alkylrest darstellt, R₂ und R₃ Methyl sind und R₄ Wasserstoff bedeutet.

8. Verbindung nach Anspruch 6 oder 7,
**dadurch gekennzeichnet,**
**dass** R₇ einen Hydroxy- oder Methoxyrest darstellt.

9. Verbindung nach einem der Ansprüche 6 bis 8,
**dadurch gekennzeichnet,**
**dass** R₆ eine Nitrosogruppe darstellt.

10. Verbindung nach einem der Ansprüche 6 bis 7,
**dadurch gekennzeichnet,**
**dass** R₆ eine Formyl- oder eine Hydroxymethylgruppe darstellt.

11. Verbindung nach einem der Ansprüche 6 bis 10,
**dadurch gekennzeichnet,**
**dass** X den Rest -NR₁₁R₁₂ darstellt, wobei die Reste R₁₁ und R₁₂ wie in Anspruch 6 definiert sind.

12. Verfahren zur Herstellung von -SO₂X enthaltenden Farbstoffen der allgemeinen Formeln II bis VII 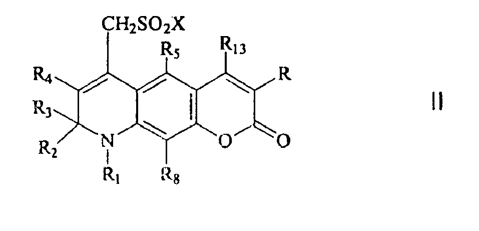 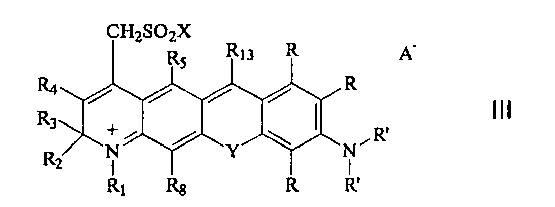 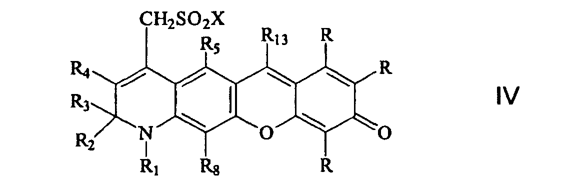 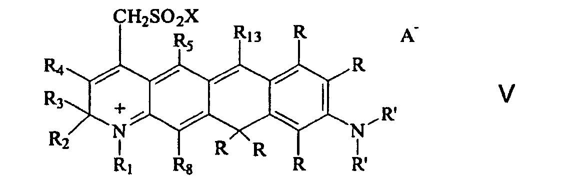 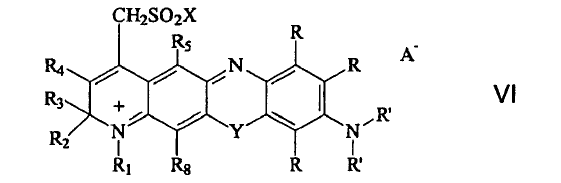 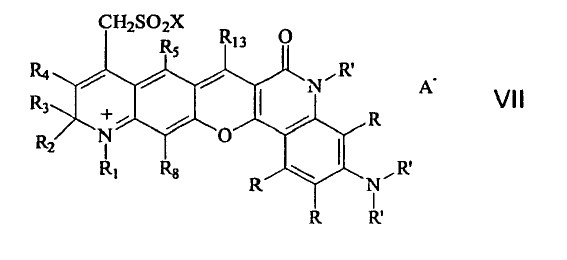 worin R₁, R₂, R₃, R₄, R₅ und R₈ wie in Ansprüchen 1 bis 11 definiert sind, R bei jedem Auftreten gleich oder verschieden sein kann und wie R₁, R₂, R₃, R₄, R₅ und R₈ definiert ist und R' bei jedem Auftreten unabhängig voneinander Wasserstoff oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, oder die Reste R und R' zusammen ein Ringsystem bilden, das eine oder mehrere Doppelbindungen enthalten kann,
R₁₃ bei jedem Auftreten unabhängig voneinander Wasserstoff oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, wobei R₁₃ insbesondere Wasserstoff, Aryl, Carboxyphenyl, Alkyl, Perfluoralkyl, Cycloalkyl, Pyridyl oder Carboxypyridyl darstellt,
X -S-R₁₀ oder -NR₁₁R₁₂ bedeutet, wobei R₁₀, R₁₁ und R₁₂ jeweils unabhängig voneinander Wasserstoff oder einen C1 bis C20 Kohlenwasserstoffrest bedeuten, welcher gegebenenfalls ein oder mehrere Heteroatome oder einen oder mehrere Substituenten enthalten kann,
Y in Formel III O, S oder Se sowie Y in Formel VI O, S oder C(R)₂ bedeutet, und
A⁻ in den Formeln III, V, VI und VII ein Anion bedeutet,
**dadurch gekennzeichnet,**
**dass** man entsprechende Verbindungen der Formeln II' bis VII' 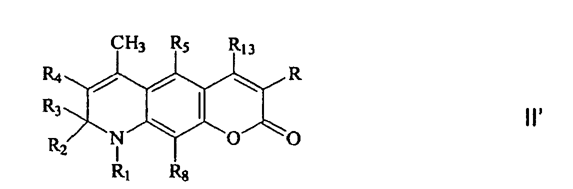 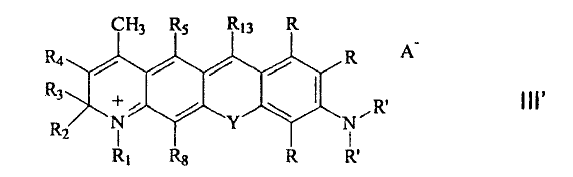 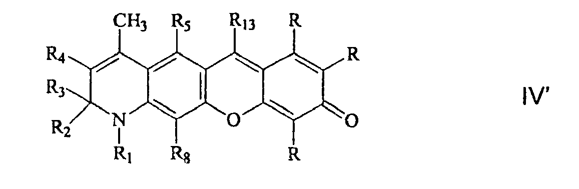 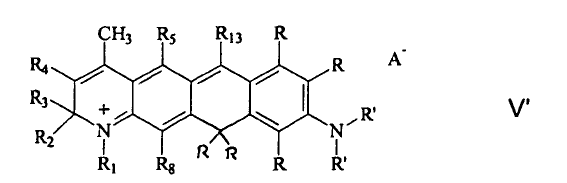 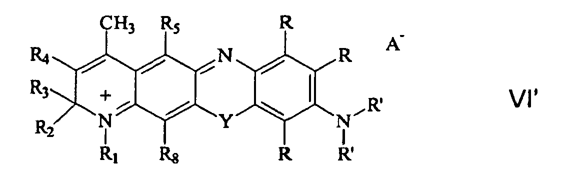 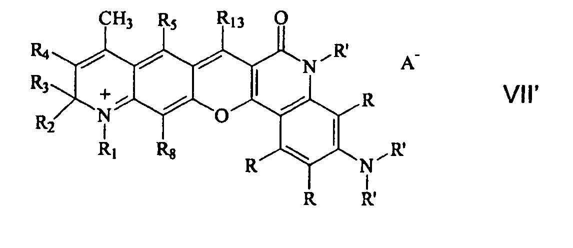 sulfoniert.

13. Verwendung einer Verbindung nach einem der Ansprüche 6 bis 11 oder erhältlich gemäß dem Verfahren nach einem der Ansprüche 1 bis 5 zur Herstellung von polycyclischen Farbstoffen.

14. Verwendung nach Anspruch 13 zur Herstellung von polycyclischen Farbstoffen der Formeln II bis VII.

15. Polycyclischer Farbstoff der allgemeinen Formel II bis VII 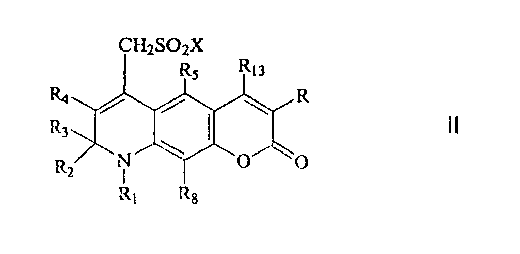 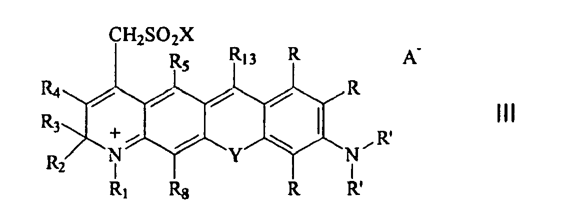 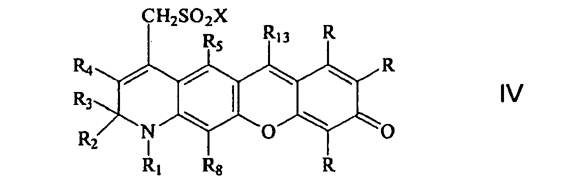 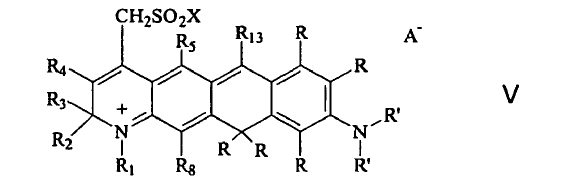 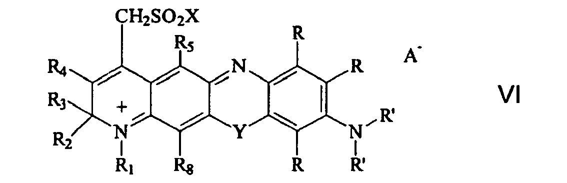 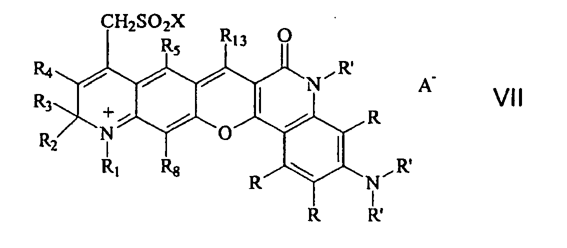 worin
R₁, R₂, R₃, R₄, R₅ und R₈ wie in den Ansprüchen 1 bis 11 definiert sind,
R' Wasserstoff oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann,
R bei jedem Auftreten unabhängig voneinander Wasserstoff, Halogen, eine Hydroxy-, Amino-, Sulfo-, Carboxy- oder Aldehydgruppe oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, oder die Reste R' und R zusammen ein Ringsystem bilden, das eine oder mehrere Mehrfachbindungen enthalten kann,
R₁₃ bei jedem Auftreten unabhängig voneinander Wasserstoff oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, wobei R₁₃ insbesondere Wasserstoff, Aryl, Carboxyphenyl, Alkyl, Perfluoralkyl, Cycloalkyl, Pyridyl oder Carboxypyridyl darstellt,
X -S-R₁₀ oder -NR₁₁R₁₂ bedeutet, wobei R₁₀, R₁₁ und R₁₂ jeweils unabhängig voneinander Wasserstoff oder einen C1 bis C20 Kohlenwasserstoffrest bedeuten, welcher gegebenenfalls ein oder mehrere Heteroatome oder einen oder mehrere Substituenten enthalten kann,
Y in Formel III O, S, oder Se sowie Y in Formel VI O, S oder C(R)₂ darstellt, und
A⁻ in den Formeln III, V, VI und VII ein Anion bedeutet.

16. Polycyclischer Farbstoff nach Anspruch 15,
**dadurch gekennzeichnet,**
**dass** X für den Rest -NR₁₁R₁₂ steht, wobei die Reste R₁₁ und R₁₂ wie in Anspruch 15 definiert sind.

17. Polycyclischer Farbstoff nach Anspruch 16,
**dadurch gekennzeichnet,**
**dass** R₁₁ oder/und R₁₂ einen mit -COOH substituierten Alkyl- oder Arylrest darstellt.

18. Verwendung eines Farbstoffs nach einem der Ansprüche 15 bis 17 zur Markierung eines Analyten.

19. Verwendung nach Anspruch 18,
**dadurch gekennzeichnet,**
**dass** es sich bei dem Analyten um ein Biomolekül, insbesondere um ein Peptid oder ein Nukleotid, handelt.

20. Verwendung nach Anspruch 18 oder 19,
**dadurch gekennzeichnet,**
**dass** die Markierung durch Bindung des Farbstoffs an eine NH₂- oder SH-Gruppe des Analyten erfolgt.

21. Verwendung eines Farbstoffes nach einem der Ansprüche 15 bis 17 zur Kopplung an einen weiteren Farbstoff.

22. Verwendung nach Anspruch 21,
**dadurch gekennzeichnet,**
**dass** die Kopplung über eine Aminogruppe des weiteren Farbstoffs erfolgt und **dadurch** ein FRET-Pärchen gebildet wird.

## Claims

1. A method for producing dihydroquinoline compounds of the general formula Ia or tetrahydroquinoline compounds of the general formula Ib 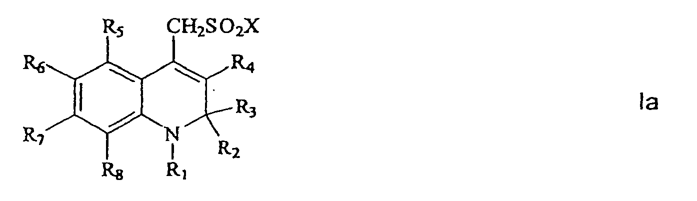 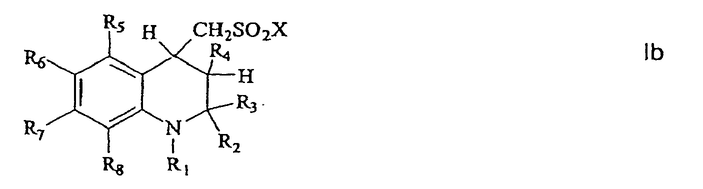 in which R₁ means a hydrocarbon group with 1-20 C atoms, wherein the hydrocarbon group may optionally contain one or more heteroatoms and/or one or more substituents,
R₂, R₃, R₄, R₅, R₆, R₇ and R₈ on each occurrence mutually independently mean hydrogen, halogen, a hydroxy, amino, sulfo, carboxy or aldehyde group or a hydrocarbon group with 1-20 C atoms, wherein the hydrocarbon group may optionally contain one or more heteroatoms and/or one or more substituents, or the residues R₁ and R₈ together form a ring system and
X means -S-R₁₀ or -NR₁₁R₁₂, wherein R₁₀, R₁₁ and R₁₂ mutually independently mean hydrogen or a C1 to C20 hydrocarbon residue, which may optionally contain one or more heteroatoms and/or one or more substituents, **characterised in that** the corresponding compounds I'a 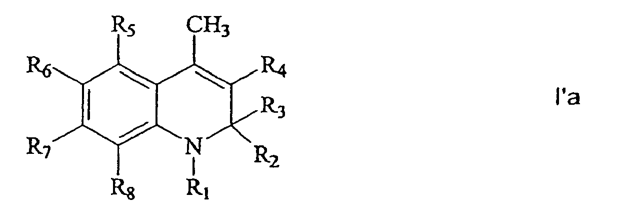 are sulfonated to yield Ia (X = OH) and optionally converted by hydrogenation to yield Ib (X = OH).

2. A method according to claim 1, **characterised in that** sulfonation is carried out by means of concentrated sulfuric acid.

3. A method according to claim 1 or claim 2, **characterised in that** the sulfonic acid group formed on sulfonation is derivatised.

4. A method according to claim 3, **characterised in that** the sulfonic acid group is converted into sulfochloride.

5. A method according to claim 3 or claim 4, **characterised in that** the sulfochloride group is converted into sulfonamide with a primary or secondary amine.

6. A dihydroquinoline compound of the general formula Ia or a tetrahydroquinoline compound of the general formula Ib 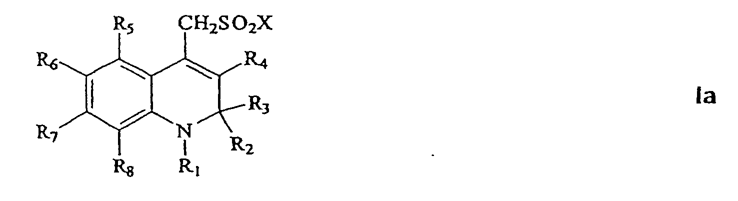 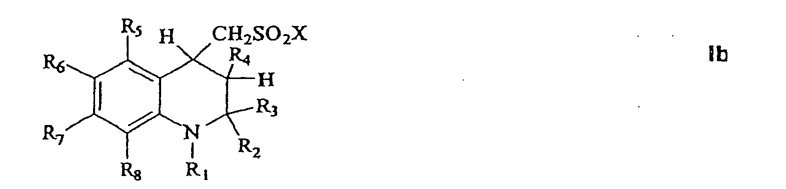 in which R₁ means a hydrocarbon group with 1-20 C atoms, wherein the hydrocarbon group may optionally contain one or more heteroatoms and/or one or more substituents,
R₂, R₃, R₄, R₅, R₆, R₇ and R₈ on each occurrence mutually independently mean hydrogen, halogen, a hydroxy, amino, sulfo, carboxy or aldehyde group or a hydrocarbon group with 1-20 C atoms, wherein the hydrocarbon group may optionally contain one or more heteroatoms and/or one or more substituents, or the residues R₁ and R₈ together form a ring system and
X means -S-R₁₀ or -NR₁₁R₁₂, wherein R₁₀, R₁₁ and R₁₂ in each case mutually independently mean hydrogen or a C1 to C20 hydrocarbon residue, which may optionally contain one or more heteroatoms and/or one or more substituents, in particular -SO₃H, -PO₃H₂ and -COOH.

7. A compound according to claim 6, in which R₁ represents an aryl or alkyl residue, in particular a C5 to C15 aryl or a C1 to C20 alkyl residue, R₂ and R₃ are methyl and R₄ means hydrogen.

8. A compound according to claim 6 or claim 7, **characterised in that** R₇ represents a hydroxy or methoxy residue.

9. A compound according to any one of claims 6 to 8, **characterised in that** R₆ represents a nitroso group.

10. A compound according to either one of claims 6 to 7, **characterised in that** R₆ represents a formyl or a hydroxymethyl group.

11. A compound according to any one of claims 6 to 10, **characterised in that** X represents the residue -NR₁₁R₁₂, wherein the residues R₁₁ and R₁₂ are as defined in claim 6.

12. A method for producing dyes containing -SO₂X of the general formulae II to VII 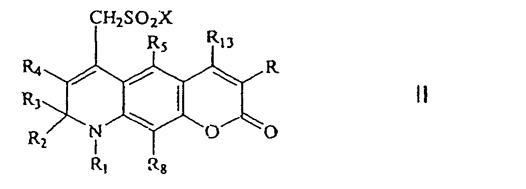 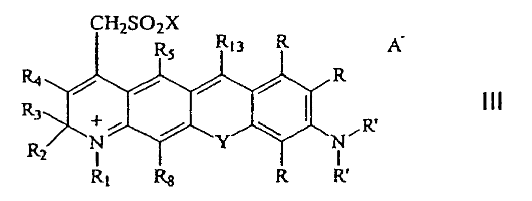 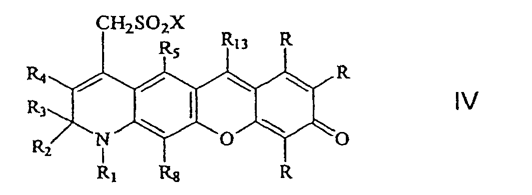 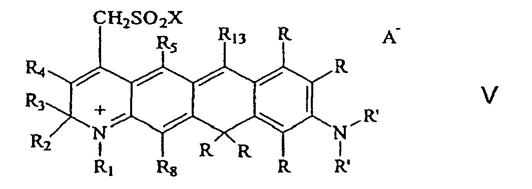 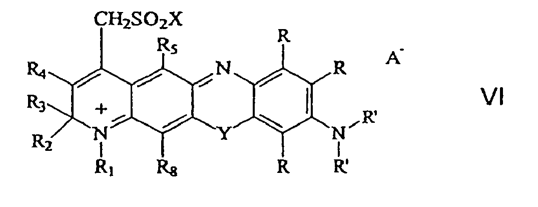 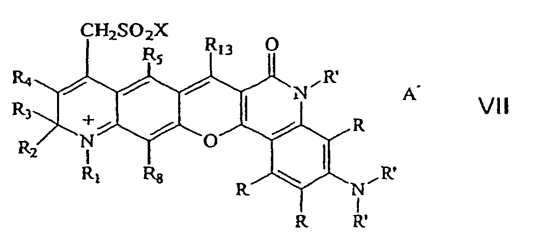 in which R₁, R₂, R₃, R₄, R₅ and R₈ are as defined in claims 1 to 11, R on each occurrence may be identical or different and is defined as R₁, R₂, R₃, R₄, R₅ and R₈ and R' on each occurrence mutually independently means hydrogen or a hydrocarbon group with 1-20 C atoms, wherein the hydrocarbon group may optionally contain one or more heteroatoms and/or one or more substituents, or the residues R and R' together form a ring system which may contain one or more double bonds,
R₁₃ on each occurrence mutually independently means hydrogen or a hydrocarbon group with 1-20 C atoms, wherein the hydrocarbon group may optionally contain one or more heteroatoms and/or one or more substituents, wherein R₁₃ in particular represents hydrogen, aryl, carboxyphenyl, alkyl, perfluoroalkyl, cycloalkyl, pyridyl or carboxypyridyl,
X means -S-R₁₀ or -NR₁₁R₁₂, wherein R₁₀, R₁₁ and R₁₂ in each case mutually independently mean hydrogen or a C1 to C20 hydrocarbon residue, which may optionally contain one or more heteroatoms and/or one or more substituents,
Y in formula III means O, S or Se and Y in formula VI means 0, S or C(R)₂, and
A⁻ in the formulae III, V, VI and VII means an anion, **characterised in that** corresponding compounds of the formulae II' to VII' 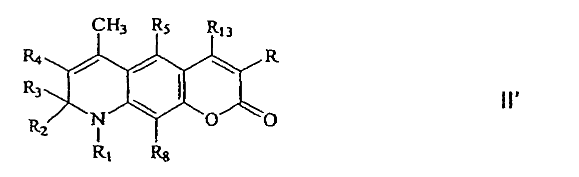 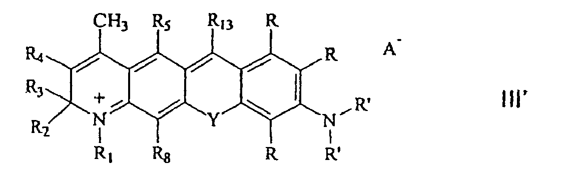 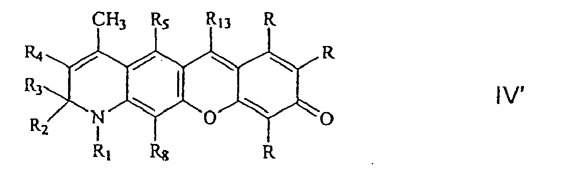 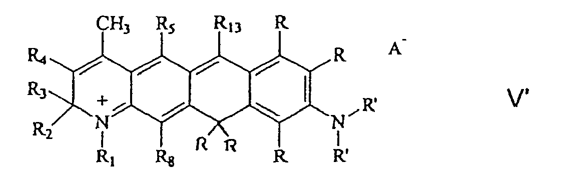 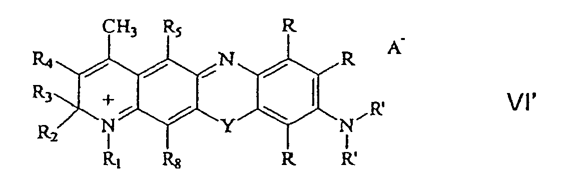 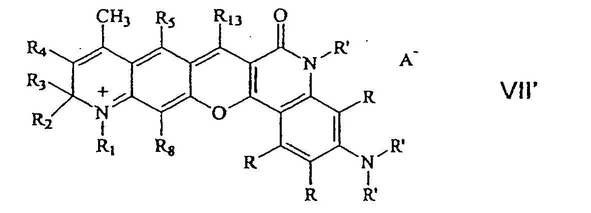 are sulfonated.

13. Use of a compound according to any one of claims 6 to 11 or obtainable by a method according to any one of claims 1 to 5 for producing polycyclic dyes.

14. Use according to claim 13 for producing polycyclic dyes of the formulae II to VII.

15. Polycyclic dye of the general formula II to VII 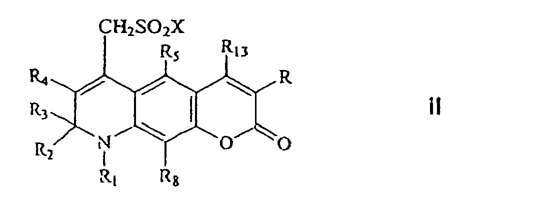 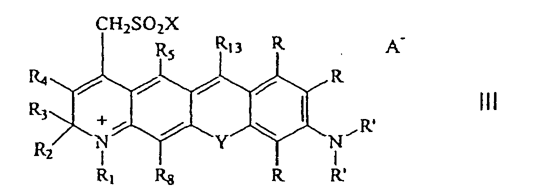 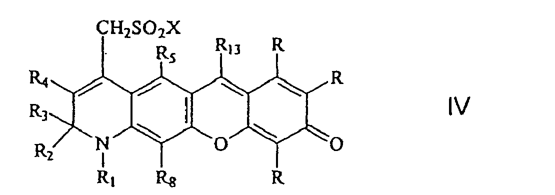 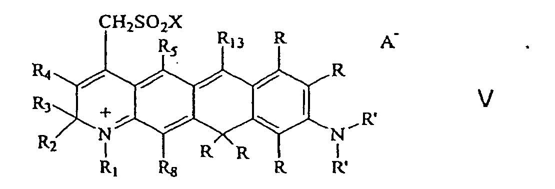 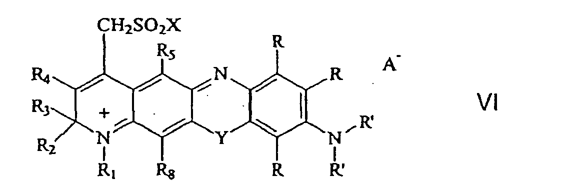 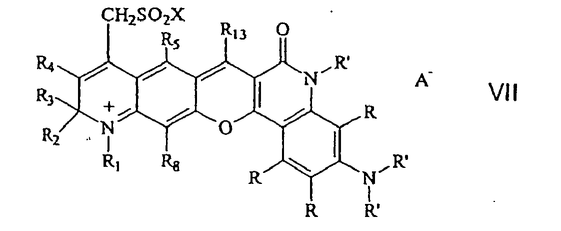 in which
R₁, R₂, R₃, R₄, R₅ and R₈ are as defined in claims 1 to 11,
R' means hydrogen or a hydrocarbon group with 1-20 C atoms, wherein the hydrocarbon group may optionally contain one or more heteroatoms and/or one or more substituents,
R on each occurrence mutually independently means hydrogen, halogen, a hydroxy, amino, sulfo, carboxy or aldehyde group or a hydrocarbon group with 1-20 C atoms, wherein the hydrocarbon group may optionally contain one or more heteroatoms and/or one or more substituents, or the residues R' and R together form a ring system which may contain one or more multiple bonds,
R₁₃ on each occurrence mutually independently means hydrogen or a hydrocarbon group with 1-20 C atoms, wherein the hydrocarbon group may optionally contain one or more heteroatoms and/or one or more substituents, wherein R₁₃ in particular represents hydrogen, aryl, carboxyphenyl, alkyl, perfluoroalkyl, cycloalkyl, pyridyl or carboxypyridyl,
X means -S-R₁₀ or ,-NR₁₁R₁₂, wherein R₁₀, R₁₁ and R₁₂ in each case mutually independently mean hydrogen or a C1 to C20 hydrocarbon residue, which may optionally contain one or more heteroatoms and/or one or more substituents,
Y in formula III represents 0, S, or Se and Y in formula VI represents 0, S or C(R)₂, and
A⁻ in the formulae III, V, VI and VII means an anion.

16. A polycyclic dye according to claim 15, **characterised in that** X denotes the residue -NR₁₁R₁₂, wherein the residues R₁₁ and R₁₂ are as defined in claim 15.

17. A polycyclic dye according to claim 16, **characterised in that** R₁₁ and/or R₁₂ represent an alkyl or aryl residue substituted with -COOH.

18. Use of a dye according to any one of claims 15 to 17 for labelling an analyte.

19. Use according to claim 18, **characterised in that** the analyte is a biomolecule, in particular a peptide or a nucleotide.

20. Use according to claim 18 or claim 19, **characterised in that** labelling proceeds by binding of the dye to an NH₂ or SH group of the analyte.

21. Use of a dye according to any one of claims 15 to 17 for coupling to a further dye.

22. Use according to claim 21, **characterised in that** coupling proceeds via an amino group of the further dye and, in this manner, a FRET pair is formed.

## Revendications

1. Procédé de production de composés de dihydroquinoline de formule générale Ia ou de composés de tétrahydroquinoline de formule générale Ib 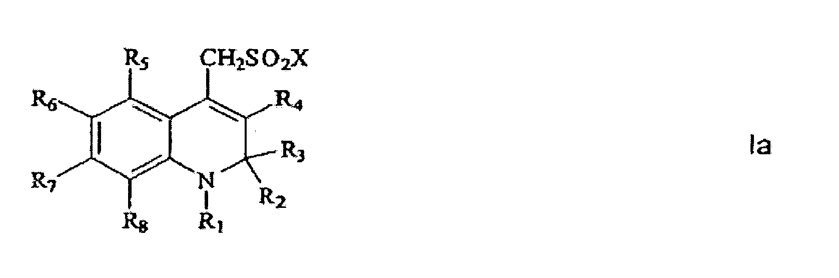 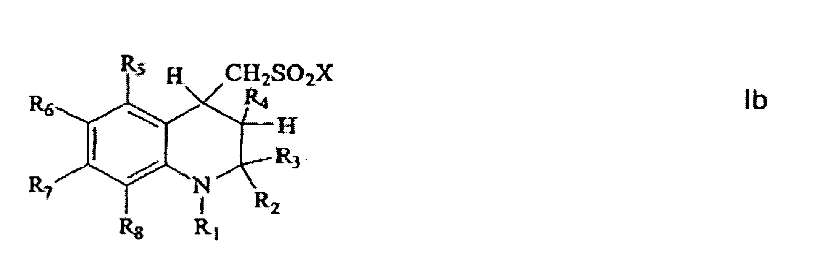 dans lesquelles R₁ désigne un groupe hydrocarbure comportant 1 à 20 atomes de carbone, le groupe hydrocarbure pouvant contenir éventuellement un ou plusieurs hétéroatomes et/ou un ou plusieurs substituants,
R₂, R₃, R₄, R₅, R₆, R₇ et R₈ désignant à chaque occurrence, indépendamment les unes des autres, un atomes d'hydrogène, d'halogène, un groupe hydroxy, amino, sulfo, carboxy ou aldéhyde ou un groupe hydrocarbure comportant 1 à 20 atomes de carbone, le groupe hydrocarbure pouvant contenir éventuellement un ou plusieurs hétéroatomes et/ou un ou plusieurs substituants, ou les radicaux R₁ et R₈ forment conjointement un système cyclique et
X désigne -S-R₁₀ ou -NR₁₁R₁₂, où R₁₀, R₁₁ et R₁₂ désignent indépendamment les uns des autres, un atome d'hydrogène ou un radical hydrocarbure en C₁ à C₂₀ qui peut contenir éventuellement un ou plusieurs hétéroatomes et/ou un ou plusieurs substituants,
**caractérisé en ce que**
l'on sulfone les composés 1'a correspondants 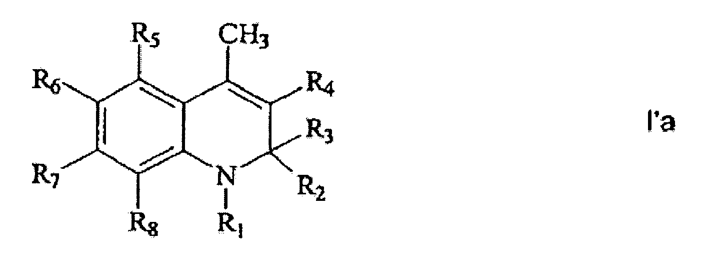 en composé Ia (X = OH) et on les convertit éventuellement en Ib (X = OH) par hydrogénation.

2. Procédé selon la revendication 1
**caractérisé en ce que**
l'on réalise la sulfonation au moyen d'acide sulfurique concentré.

3. Procédé selon la revendication 1 ou 2,
**caractérisé en ce que**
l'on dérivatise lors de la sulfonation, les groupes d'acide sulfonique formés.

4. Procédé selon la revendication 3
**caractérisé en ce que**
l'on convertit le groupe d'acide sulfonique en sulfochlorure.

5. Procédé selon la revendication 3 ou 4,
**caractérisé en ce que**
l'on convertit le groupe sulfochlorure comportant une amine primaire ou secondaire, en sulfonamide.

6. Composé de dihydroquinoline de formule générale Ia ou composé de tétrahydroquinoline de formule générale Ib 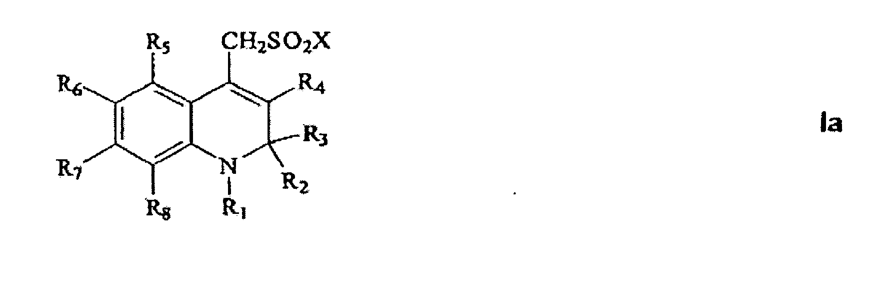 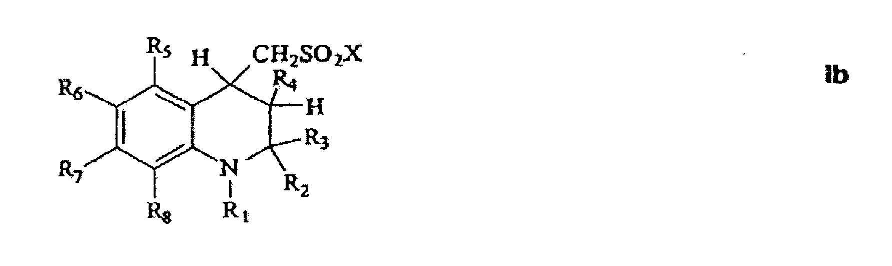 dans lesquelles R₁ désigne un groupe hydrocarbure comportant 1 à 20 atomes de carbone, le groupe hydrocarbure pouvant contenir éventuellement un ou plusieurs hétéroatomes et/ou un ou plusieurs substituants
R₂, R₃, R₄, R₅, R₆, R₇ et R₈ désignant à chaque occurrence, indépendamment les unes des autres, un atomes d'hydrogène, d'halogène, un groupe hydroxy, amino, sulfo, carboxy ou aldéhyde ou un groupe hydrocarbure comportant 1 à 20 atomes de carbone, le groupe hydrocarbure pouvant contenir éventuellement un ou plusieurs hétéroatomes et/ou un ou plusieurs substituants, ou les radicaux R₁ et R₈ forment conjointement un système cyclique et
X désigne -S-R₁₀ ou -NR₁₁R₁₂, où R₁₀, R₁₁ et R₁₂ désignent indépendamment les uns des autres, un atome d'hydrogène ou un radical hydrocarbure en C₁ à C₂₀ qui peut contenir éventuellement un ou plusieurs hétéroatomes et/ou un ou plusieurs substituants, en particulier -SO₂H, -PO₃H₂ et -COOH.

7. Composé selon la revendication 6
dans lequel R₁ représente un radical aryle ou alkyle, en particulier un radical aryle en C₅ à C₁₅ ou un radical alkyle en C₁ à C₂₀, R₂ et R₃ désignent un groupe méthyle et R₄, un atome d'hydrogène.

8. Composé selon la revendication 6 ou 7,
**caractérisé en ce que**
R₇ est un radical hydroxy ou méthoxy.

9. Composé selon l'une des revendications 6 à 8,
**caractérisé en ce que**
R₆ représente un groupe nitroso.

10. Composé selon l'une des revendications 6 à 7,
**caractérisé en ce que**
R₆ représente un groupe formyle ou un groupe hydroxyméthyle.

11. Composé selon l'une des revendications 6 à 10,
**caractérisé en ce que**
X représente le radical -NR₁₁R₁₂, les radicaux R₁₁ et R₁₂ étant tels que définis à la revendication 6.

12. Procédé de production de colorants contenant -SO₂X de formules générales II à VII 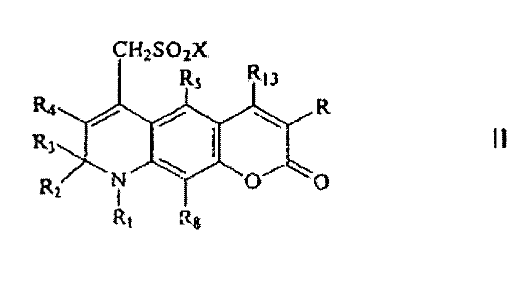 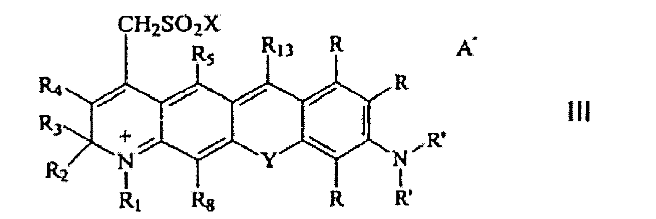 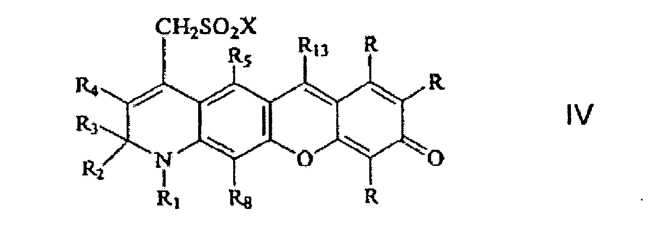 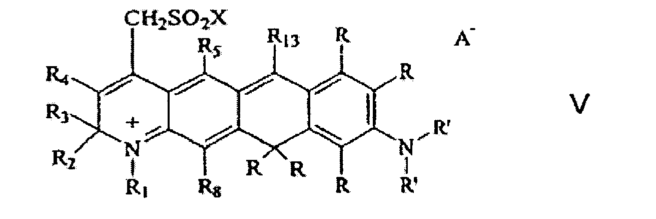 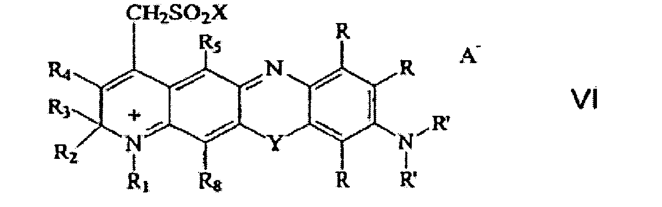 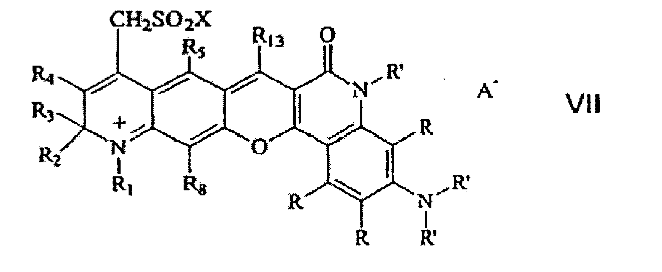 dans lesquelles
R₁, R₂, R₃, R₄, R₅ et R₆ sont tels que définis dans les revendications 1 à 11,
R à chaque occurrence, peut être identique ou différent et est défini tel que R₁, R₂, R₃, R₄, R₅ et R₆ et les R' à chaque occurrence, indépendamment les uns des autres, désignent un atome d'hydrogène ou un groupe hydrocarbure comportant 1 à 20 atomes de carbone, le groupe hydrocarbure pouvant contenir éventuellement un ou plusieurs hétéroatomes et/ou un ou plusieurs substituants, ou les radicaux R et R' forment conjointement un système cyclique qui peut contenir une ou plusieurs doubles liaisons,
les R₁₃ à chaque occurrence, désignent indépendamment les uns des autres, un atome d'hydrogène ou un groupe hydrocarbure comportant 1 à 20 atomes de carbone, le groupe hydrocarbure pouvant contenir éventuellement un ou plusieurs hétéroatomes et/ou un ou plusieurs substituants, R₁₃ représentant en particulier un atome d'hydrogène, un groupe aryle, carboxyphényle, alkyle, perfluoroalkyle, cycloalkyle, pyridyle ou carboxypyridyle,
X désigne -SR₁₀ ou (NR₁₁R₁₂, R₁₀, R₁₁, et R₁₂ désignant respectivement, indépendamment les uns des autres, un atome d'hydrogène ou un radical hydrocarbure en C₁ à C₂₀ qui peut contenir éventuellement un ou plusieurs hétéroatomes ou un ou plusieurs substituants,
Y désigne, dans la formule III, O, S ou Se et Y désigne, dans la formule VI, O, S ou C(R)₂, et
A⁻ dans les formules III, V, VI et VII désigne un anion,
**caractérisé en ce que**
l'on sulfone les composés correspondants de formules II' à VII'

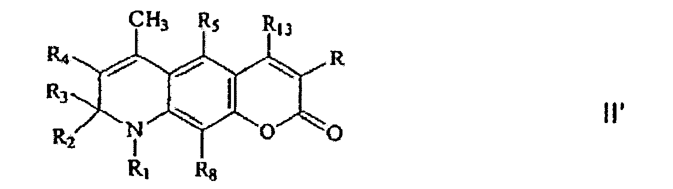

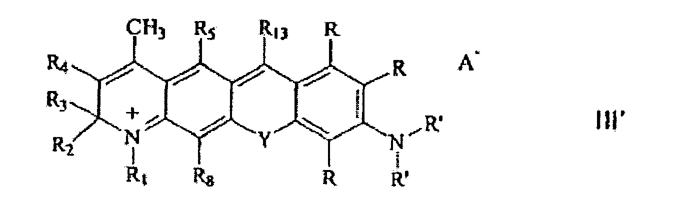

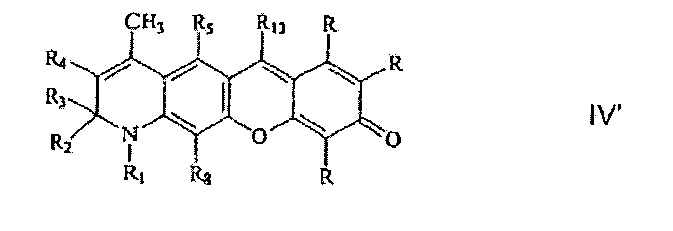

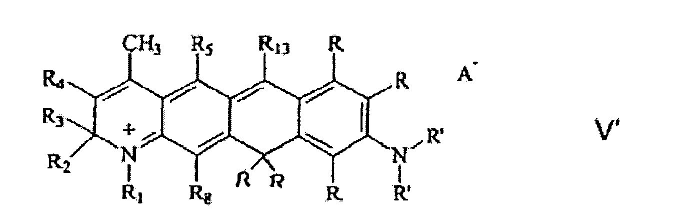

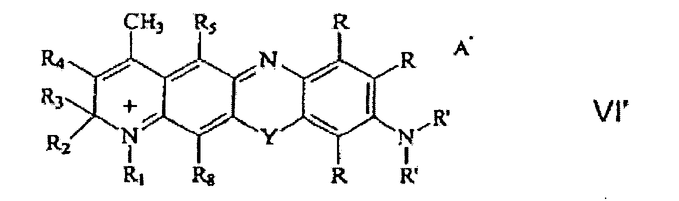

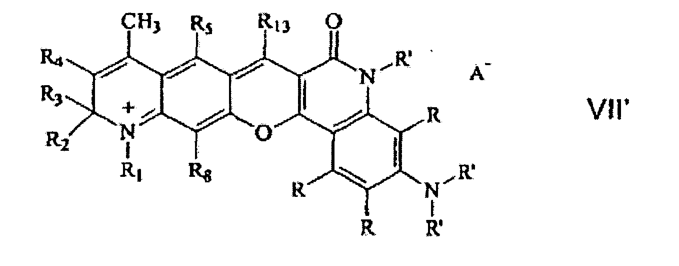

13. Utilisation d'un composé selon l'une des revendications 6 à 11 ou pouvant être obtenu selon le procédé selon l'une des revendications 1 à 5, pour la production de colorants polycycliques.

14. Utilisation selon la revendication 13, pour la production de colorants polycycliques de formules II à VII.

15. Colorant polycyclique de formule générale II à VII 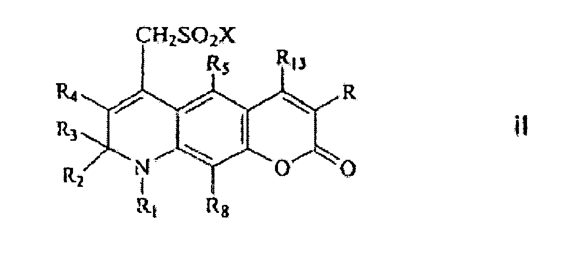 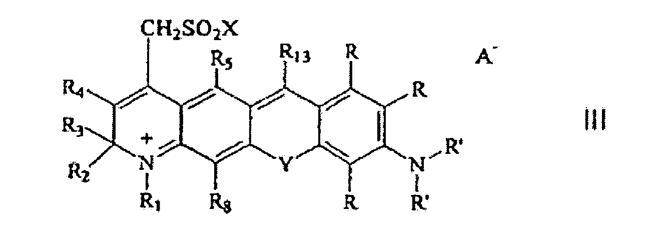 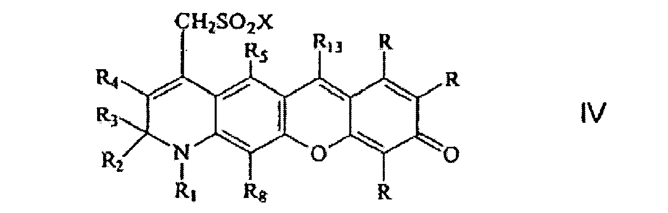 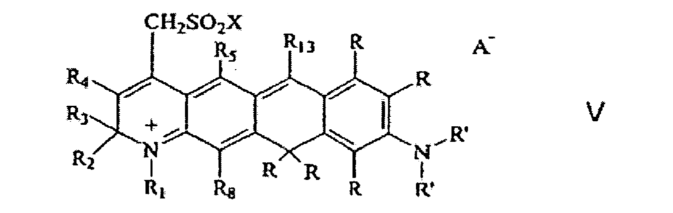 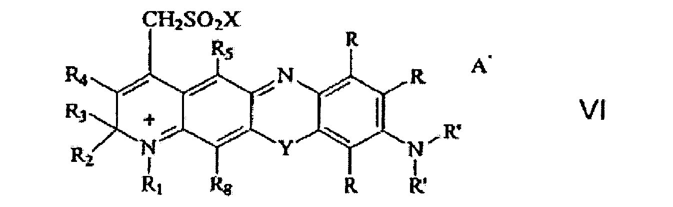 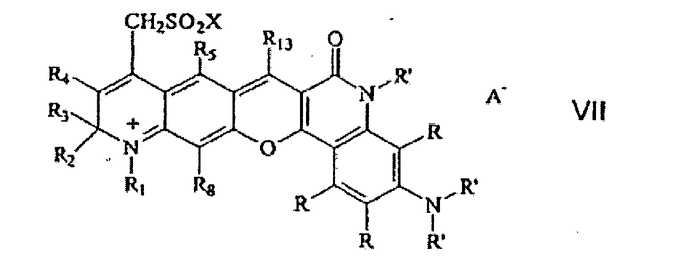 dans lesquelles
R₁, R₂, R₃, R₄, R₅ et R₆ sont tels que définis dans les revendications 1 à 11,
R' désigne un atome d'hydrogène ou un groupe hydrocarbure comportant 1 à 20 atomes de carbone, le groupe hydrocarbure pouvant contenir éventuellement un ou plusieurs hétéroatomes et/ou un ou plusieurs substituants,
les R, à chaque occurrence, indépendamment les uns des autres, désignent un groupe hydrocarbure, un atome d'halogène, un groupe hydroxy, amino, sulfo, carboxy ou aldéhyde ou un groupe hydrocarbure comportant 1 à 20 atomes de carbone, le groupe hydrocarbure pouvant contenir éventuellement un ou plusieurs hétéroatomes et/ou un ou plusieurs substituants ou les radicaux R' et R forment conjointement un système cyclique qui peut contenir un ou plusieurs composés multiples,
les R₁₃, à chaque occurrence, désignent indépendamment les uns des autres, un atome d'hydrogène ou un groupe hydrocarbure comportant 1 à 20 atomes de carbone, le groupe hydrocarbure pouvant contenir éventuellement un ou plusieurs hétéroatomes et/ou un ou plusieurs substituants, R₁₃ désignant en particulier un atome d'hydrogène, un groupe aryle, carboxyphényle, alkyle, perfluoroalkyle, cycloalkyle, pyridyle ou carboxypyridyle,
X désigne -S-R₁₀ ou NR₁₁R₁₂, R₁₀, R₁₁ et R₁₂ désignant respectivement, indépendamment les uns des autres, un atome d'hydrogène ou un radical hydrocarbure en C₁ à C₂₀ qui peut éventuellement contenir un ou plusieurs hétéroatomes ou un ou plusieurs substituants,
Y dans la formule III, représente O, S ou Se et Y dans la formule VI, représente O, S ou C(R)₂, et
A⁻ dans les formules III, V, VI et VII désigne un anion.

16. Colorant polycyclique selon la revendication 15,
**caractérisé en ce que**
X représente le radical -NR₁₁R₁₂, les radicaux R₁₁ et R₁₂ étant tels que définis dans la revendication 15.

17. Colorant polycyclique selon la revendication 16,
**caractérisé en ce que**
R₁₁ et/ou R₁₂ représentent un radical alkyle ou aryle substitué par -COOH.

18. Utilisation d'un colorant selon l'une des revendications 15 à 17, pour le marquage d'un analyte.

19. Utilisation selon la revendication 18,
**caractérisée en ce que**
l'analyte est une biomolécule, en particulier un peptide ou un nucléotide.

20. Utilisation selon la revendication 18 ou 19,
**caractérisée en ce que**
le marquage s'effectue par liaison du colorant à un groupe NH₂- ou SH-.

21. Utilisation d'un colorant selon l'une des revendications 15 à 17, pour le couplage à un autre colorant.

22. Utilisation selon la revendication 21,
**caractérisée en ce que**
le couplage s'effectue par un groupe amino de l'autre colorant et ainsi est formée une paire de FRET.
